# EUROPÄISCHE PATENTSCHRIFT

(11) **EP 0 297 597 B1**
(45) Veröffentlichungstag und Bekanntmachung des Hinweises auf die Patenterteilung: **25.10.1995**
(21) Anmeldenummer: 88110523.3
(22) Anmeldetag: 01.07.1988
(51) Int. Cl.: C12Q 1/56, C12Q 1/37

(54) **Verfahren zur Bestimmung der Aktivität von Serinproteasen oder Serinproteaseninhibitoren**
Method for determining the activity of serine proteinases or serine proteinase inhibitors
Méthode pour déterminer l'activité de sérine protéases ou d'inhibiteurs de sérine protéases

(30) Priorität: 03.07.1987 DE 3722082
(43) Veröffentlichungstag der Anmeldung: 04.01.1989
(73) Patentinhaber: BEHRINGWERKE Aktiengesellschaft, 35001 Marburg (DE)
(72) Erfinder: Stief, Thomas, Dr., D-3550 Marburg (DE); Heimburger, Norbert, Prof. Dr., D-3550 Marburg (DE)

(56) Entgegenhaltungen:
- EP-A- 0 198 322
- BIOCHEMISTRY, Band 25, Nr. 21, 21. Oktober 1986, American Chemical Society, Easton, US; D.A. LAWRENCE et al., Seiten 6351-6355#
- JOURNAL OF BIOLOGICAL CHEMISTRY, Band 262, Nr. 13, 05. Mai 1987, American Society of Biological Chemists, Inc., Baltimore, US; B.-H. SHIEH et al., Seiten 6055-6059#
- CLINICAL CHEMISTRY, Band 32, Nr. 3, 1986, Winston-Salem, NC (US); J. CHMIELEWSKA et al.#
- RÖMPPS CHEMIE-LEXIKON, 8. Auflage, 1979; Seite 642#

## Beschreibung

Die Erfindung bezieht sich auf ein Verfahren zum Bestimmen der Aktivität von Serinproteasen oder Serinprotease-Inhibitoren in Plasma oder anderen biologischen Flüssigkeiten.

Der Organismus verfügt über zwei im Gleichgewicht stehende Systeme, um sich sowohl vor Blutverlust als auch vor Thrombosen zu schützen: das Gerinnungssytem und das fibrinolytische System. Das Zusammenspiel zwischen beiden Systemen gewährleistet, daß zunächst zur Blutstillung unlösliche Fibrinpolymere entstehen, die während der Wundheilung durch den lytischen Vorgang der Fibrinolyse wieder abgebaut werden.

Thrombin und Plasmin stellen die Schlüsselenzyme beider Systeme dar. Unter physiologischen Bedingungen steht das dynamische Gleichgewicht zwischen dem Gerinnungs- und Fibrinolysesystem unter Kontrolle der thromboplastischen Aktivität von Thrombin und der thrombolytischen Aktivität von Plasmin. Zum Gleichgewicht tragen die jeweiligen Inhibitoren bei.

Plasmin ist eine relativ unspezifische trypsinähnliche Protease, der neben der Fibrinauflösung weitere physiologische und pathophysiologische Funktionen zugeschrieben werden. So wie auch viele der Gerinnungenzyme, zirkuliert Plasmin in Form eines inaktiven Vorläufers, des Plasminogens, im Blut, und wird erst auf bestimmte Reize hin aktiviert. Die Umwandlung von Plasminogen in Plasmin wird durch Plasminogenaktivatoren katalysiert.

Die Aktivierung des Plasminogens kann durch vier verschiedene Plasminogenaktivator-Systeme geschehen:
1. Ein Faktor XII abhängiges System,
2. einen aus Streptokokken isolierten Plasminogenaktivator, die Streptokinase,
3. Gewebe-Plasminogenaktivator (t-PA) und
4. urinären Plasminogenaktivator (u-PA bzw.Urokinase).

Physiologische Bedeutung kommt nur den beiden letztgenannten Plasminogenaktivatoren, nämlich t-PA und u-PA, zu.

Sowohl die Plasminogenaktivatoren als auch Plasmin sind typische Serinproteasen. Die verfügbare Menge aktiven Plasmins und aktiver Plasminogenaktivatoren wird im Körper u.a. durch unspezifische und spezifische Inhibitoren reguliert. So wird z.B. Plasmin unspezifisch durch alpha₂-Makroglobulin, Inter-alpha-Inhibitor, C₁-Esterase-Inhibitor und alpha₁-Antitrypsin inhibiert, außerdem spezifisch durch alpha₂-Antiplasmin; die Plasminogenaktivatoren werden unspezifisch u.a. durch Antithrombin III und alpha₂-Antiplasmin gehemmt, außerdem spezifisch durch Plasminogenaktivator-Inhibitoren.

Diese Plasminogenaktivator-Inhibitoren können mit immunologischen Methoden in mindestens drei Klassen unterteilt werden:
a) endotheliale Inhibitoren: dazu gehören die aus Endothel und Plättchen freigesetzten Inhibitoren, im folgenden PAI1 genannt;
b) placentale Inhibitoren: dazu gehören die Inhibitoren, die aus Placenta und Leukozyten freigesetzt werden, im folgenden PAI2 genannt;
c) heparinabhängige Inhibitoren: dazu gehört beispielsweise der Protein-C-Inhibitor, der PAI3 genannt wird.

Ein gut funktionierendes, sich auf die momentanen Bedürfnisse des Organismus rasch einstellendes Gleichgewicht der Komponenten des Fibrinolysesystems ist von äußerster Wichtigkeit. Eine Über- oder Unterfunktion fibrinolytischer Aktivität kann fatale Folgen haben, nämlich sowohl Blutungen als auch Thrombosen. Dabei scheint klinisch den Inhibitoren der Plasminogenaktivatoren eine besonders große Bedeutung zuzukommen, da insbesondere stark erhöhte Konzentrationen zu lebensbedrohlichen Komplikationen führen können.

Solche Zusammenhänge sind bereits bei Patienten mit tiefer Beinvenenthrombose, akutem Herzinfarkt, bakteriellem Blutbefall, Lebererkrankungen, Bauchspeicheldrüsenentzündungen, Krebsleiden sowie in der Spätphase der Schwangerschaft und bei Patientinnen mit Schwangerschaftstoxikose beschrieben worden.

Es besteht daher ein überaus großes klinisches Interesse an der Bereitstellung von zuverlässigen, schnellen Methoden zur Bestimmung verschiedener Komponeten des Fibrinolysesystems im Plasma, Serum oder anderen biologischen Flüssigkeiten von Patienten mit einer gestörten Regulation der Fibrinolyse. Jedoch war aufgrund der immer vorhandenen spezifischen oder unspezifischen Serinprotease-Inhibitoren eine rasche und exakte Bestimmung z.B. von Plasminogenaktivatoren oder Plasminogenaktivator-Inhibitoren bisher nicht möglich.

Den bisher bekannten Verfahren zur PAI-Bestimmung liegt das gemeinsame Prinzip zugrunde, die Aktivität des Inhibitors nach Zugabe einer definierten Menge eines Plasminogenaktivators, meistens t-PA, durch Bestimmen der t-PA-Restaktivität zu messen, indem Plasminogen und ein chromogenes Plasminsubstrat, dessen Umsetzung dann letztlich detektiert wird, bereitgestellt werden.

Die Genauigkeit dieser funktionellen Bestimmung der Plasminogenaktivator-Inhibitoren ist vom Eliminieren aller anderen, in den beschriebenen Reaktionsablauf möglicherweise eingreifenden Inhibitoren abhängig. Plasmin z.B. wird wie erwähnt durch mindestens fünf gut beschriebene Plasma-Proteine inhibiert. Die im Stand der Technik beschriebenen Verfahren zur Bestimmung der Plasminogenaktivator-Inhibitoren setzen deshalb entweder zusätzliche Plasmatrennungsschritte voraus, wie z.B. eine Euglobulin-Fällung, oder erfordern ein Ansäuern und anschließende Neutralisierung durch hohe Verdünnungen der Probe (Speiser et al., Thromb.Res. 44, 503-515, 1986; Chmielewska und Wiman, Clin.Chem. 32, 482-485, 1986). Beide Verfahren sind relativ zeitaufwendig und methodisch anspruchsvoll.

Aufgabe der vorliegenden Erfindung war es daher, ein Verfahren zur Verfügung zu stellen, mit dem die Konzentration von Serinproteasen und Serinprotease-Inhibitoren in Plasma, Serum oder anderen biologischen Flüssigkeiten schnell und zuverlässig bestimmt werden kann.

Diese Aufgabe wird erfindungsgemäß dadurch gelöst, daß man einer auf den Gehalt an Serinproteasen oder Serinprotease-Inhibitoren zu untersuchenden Probe mindestens ein Oxidationsmittel und gegebenenfalls mindestens ein Detergenz zusetzt.

Es hat sich überraschenderweise gezeigt, daß der Zusatz eines geeigneten Oxidationsmittels, gegebenenfalls in Verbindung mit einem Detergenz in Abhängigkeit von den Untersuchungsbedingungen die störungsfreie Bestimmung der Aktivität entweder der Plasminogenaktivator-Inhibitoren, des Gewebe- und des urinären Plasminogenaktivators, des Thrombins, der Elastase oder des Plasminogens erlauben. Die exakte Bestimmung der genannten Proteine setzt die quantitative Inaktiverung spezifischer und unspezifischer Plasmin-Inhibitoren voraus. Überraschenderweise wird unter den erfindungsgemäßen Bedingungen auch alpha₂-Antiplasmin nahezu vollständig inaktiviert, während z.B. die Plasminogenaktivator-Inhibitoren, Plasminogenaktivatoren und Plasmin aktiv bleiben. Dieses Ergebnis war vollkommen überraschend, weil im Stand der Technik Lawrence und Loskutoff (Biochemistry 25, 6351-6355, 1986) berichten, daß sie unter ihren Testbedingungen eine Inaktivierung der Plasminogenaktivator-Inhibitoren durch Wasserstoffperoxid in Konzentrationen von ca. 10 mM/l (mM=mmol) und Chloramin-T in einer Konzentration von ungefähr 10 uM/l beobachten. Dieser Effekt entspricht insofern den Erwartungen, als die Plasminogenaktivator-Inhibitoren zu der Familie der Serinprotease-Inhibitoren (Serpins) gehören, deren Archetypus, nämlich alpha₁-Protease-Inhibitor, im aktiven Zentrum ein oxidierbares Methionin enthält. Oxidation dieses Methionins führt zu einem Methioninsulfoxid, mit dessen Bildung die inhibitorischen Funktionen verloren gehen. Lawrence und Loskutoff zeigen sowohl für alpha₁-Protease-Inhibitor als auch für Plasminogenaktivator-Inhibitor die Reversibilität der Inaktivierung durch Behandlung mit Dithiothreitol und Methionin-Sulfoxid-Peptid-Reduktase, d.h., unter den dort beschriebenen Bedingungen wird der Plasminogenaktivator-Inhibitor nachweislich durch die Oxidation inaktiviert. Umsoweniger konnte erwartet werden, daß die in der Reaktion entstehenden Komplexe aus Plasminogenaktivator und Plasminogenaktivator-Inhibitor auch unter den erfindungsgemäßen oxidierenden Bedingungen erhalten bleiben. Überraschend ist, daß die erfindungsgemäßen Bedingungen zu einer Inaktivierung von alpha₂-Antiplasmin führt, obwohl kürzlich der Nachweis der Oxidationsresistenz dieses Inhibitors erbracht wurde (Shieh and Travis, J.Biol.Chem. 262, 6055-6059, 1987). Darüberhinaus ist es höchst bemerkenswert, daß die Reaktion nicht inhibierten Plasminogenaktivators mit seinem Substrat, einem natürlichen oder synthetischen Plasminogen, sowie die davon abhängigen enzymatischen Reaktionen während des Oxidationsprozesses ungestört ablaufen.

Bevorzugte erfindungsgemäß zuzusetzende Oxidationsmittel sind Reagenzien, welche vorzugsweise bei pH 7.5-8.8 Methionin zu Methioninsulfoxid oxidieren. Nach Schechter et al. (Biochem. 14, 4497-4503, 1975) gehören hierzu Halogenoamine und -imide. Jedoch wären auch Substanzen aus der Gruppe der reaktiven Halogene wie Halogenoamide, -imine oder -hydroxyle einschließlich deren Salze geeignet. Erfindungsgemäße Oxidationsmittel sind Wasserstoffperoxid, Salze der Peroxomonoschwefelsäure oder der Peroxodischwefelsäure, oxidiertes Glutathion, Chloramin T, Chloramin B, Salze der HOCl oder N-Chlorsuccinimid. Besonders bevorzugt sind Chloramine oder Salze der HOCl, entsprechend den natürlichen Sekretionsprodukten aktivierter Makrophagen oder polymorphkerniger Neutrophiler (Weiss et al. Science 222, 625-628, 1983).

Zur Umwandlung von Methionin in Methioninsulfoxid eignen sich nach Savige und Fontana (Methods in Enzymology 47, 453 - 459, 1977) Farbstoff- (z. B. Methylenblau, Eosin, Riboflavin und besonders spezifisch durch Hematoporphyrin bei pH unter 7) vermittelte Photooxidation sowie Substanzen, die positives Halogen enthalten, wie N-Bromsuccinimid; N-Chlorsuccinimid; 2,4,6-Tribromo-4-methylcyclohexadienon; BNPS-Skatol (2-(nitrophenylsulfonyl)-3-methyl-3-bromoindolamin); Chloramin T; t-Butylhypochlorit; Trichlormethansulfonylchlorid, ferner 1-Chlorobenzotriazol, Jodobenzenedichlorid in wäßrigem Pyridin, Pyridinbromidkomplexe, Quinoline und besonders 1,4-Diazobicyclo (2.2.2) octane in wäßriger Essigsäure.

Obwohl unter den von Lawrence und Loskutoff beschriebenen Bedingungen die Verwendung dieser Oxidationsmittel zu einer Inaktivierung des Plasminogenaktivator-Inhibitors führt, konnte im hier beschriebenen erfindungsgemäßen Testsystem der Erhalt der PAI-Aktivität im PAI/PA-Komplex gezeigt werden.

Zweckmäßigerweise wird für die Oxidation Chloramin T eingesetzt, und zwar in einer Konzentration zwischen 0,1 mM/l bis 25 mM/l. Als besonders bevorzugt hat sich die Verwendung von 0,5-4,5 mM/l Chloramin T erwiesen.

Das Ausmaß der oxidierenden Wirkung von Chloramin T ist von der Art des zugesetzten Detergenz abhängig. Als besonders zweckmäßig hat sich die Verwendung von Polyoxyethylenäther, z.B. ^{R}Triton x 100, erwiesen. Durch den Tritonzusatz sollen einerseits die störenden Inhibitoren, wie alpha₂-Antiplasmin und Antithrombin III, so weit denaturiert werden, daß ihr reaktives Zentrum der Oxidation zugänglich wird, andererseits muß jedoch die Aktivität der am Nachweis beteiligten Enzyme, also der Plasminogenaktivatoren und des Plasmins, erhalten bleiben. Die Tritonkonzentration sollte deshalb zwischen 0,005 und 0,5 Gew.%, bevorzugt 0,03-0,15 Gew.%, betragen.

Die erfindungsgemäß zugesetzten omega-Aminocarbonsäuren bewirken einerseits, daß das Plasmin sehr viel langsamer durch alpha₂-Antiplasmin inhibiert wird, so daß alpha₂-Antiplasmin wirkungsvoller durch Oxidation inaktiviert werden kann, andererseits scheint durch die Verwendung von omega-Aminocarbonsäuren, bevorzugt Lysin, epsilon-Aminocarbonsäure, Tranexamsäure oder Derivate dieser omega-Aminocarbonsäuren, die Aktivität der Urokinase stimuliert zu werden. So führt der Zusatz von Lysin zu einer Probe, deren Plasminogenaktivator-Inhibitorengehalt ohne Anwendung eines Oxidationsmittels bestimmt werden soll, im Endeffekt zu einer im Vergleich zur omega-aminocarbonsäure-freien Probe um mehr als 100% erhöhten Extinktion (s. die Vergleichsversuche 1 und 2).

Die Zugabe von omega-Aminocarbonsäuren ist von der Art des verwendeten Plasminogenaktivators abhängig. Omega-Aminocarbonsäuren blockieren natürlich vorkommenden Gewebe-Plasminogenaktivator bezüglich seiner Plasminogenaktivierung, haben aber keinen Einfluß auf die Plasminogenaktivierungkapazität von urinären Plasminogenaktivatoren oder bestimmten synthetisch oder gentechnisch hergestellten Derivaten von Gewebe-Plasminogenaktivatoren.

Für die Durchführung des erfindungsgemäßen Verfahrens hat sich die Anwendung von Lysin in Konzentrationen von 10 bis 3000 mM/l im Reaktionsansatz, besonders zweckmäßig in einer Konzentration von 20 bis 100 mM/l als bevorzugt erwiesen, genauso wie die Verwendung von epsilon-Aminocapronsäure in einer Konzentration von 2 bis 500 mM/l, bevorzugt 10-50 mM/l, oder Tranexamsäure in einer Konzentration von 0,1 bis 50 mM/l, bevorzugt 1-5 mM/l. Verwendung der omega-Aminocarbonsäuren in diesen Konzentrationen führt reproduzierbar zu einer mindesten 10fach verlangsamten Reaktion zwischen Plasmin und alpha₂-Antiplasmin.

Für die Bestimmung der Aktivität von Plasminogenaktivator-Inhibitoren muß der Probe zu Beginnn eine definierte Menge Plasminogenaktivator zugesetzt werden, um anschließend aus der Differenz zwischen dieser definierten Menge Plasminogenaktivator und der nach der Reaktion mit dem Plasminogenaktivator-Inhibitor noch nachzuweisenden Menge Plasminogenaktivator die Inhibitorkonzentration bestimmen zu können. Ebenso erfordert die Bestimmung von Plasminogen einen Überschuß an Plasminogenaktivatoren, um die Plasminogenaktivierung nicht zu limitieren. Es können dabei sowohl Gewebe-Plasminogenaktivatoren als auch urinärer Plasminogenaktivator oder ein natürliches und/oder synthetisches Derivat einer dieser Plasminogenaktivatoren oder auch eine Kombination von zwei oder mehreren Proteinen mit Plasminogenaktivatoraktivität verwendet werden. Voraussetzung ist lediglich, daß der verwendete Plasminogenaktivator mit dem zu bestimmenden Plasminogenaktivator-Inhibitor bzw. Plasminogen reagieren kann. Bei der Verwendung natürlichen Gewebe-Plasminogenaktivators sollte zweckmäßigerweise keine omega-Aminocarbonsäure zugesetzt werden, da diese die plasminogenolytische Aktivität von t-PA inhibiert. In diesem Falle sollte jedoch die Gewebe-Plasminogenaktivatoraktivität durch Zugabe von löslichem Fibrin oder Fibrinogenfragmenten, Polylysin oder polysulfatierten Polysacchariden stimuliert werden.

Das Substrat für den Plasminogenaktivator, das für die Bestimmung der Aktivität von Plasminogenaktivator-Inhibitoren und Plasminogenaktivatoren ebenfalls zur Verfügung gestellt wird, kann natürliches Plasminogen oder eines seiner natürlichen oder synthetischen Derivate sein. Das verwendete Plasminogen muß lediglich nach seiner Aktivierung entweder zu einem aktiven Plasmin führen, das in einer nachfolgenden Reaktion ein chromogenes Plasminsubstrat umsetzen kann, oder aber selbst nach der Aktivierung durch den Plasminogenaktivator zu einem meßbaren Umwandlungsprodukt führen. Bevorzugt werden Plasminogene ohne alpha₂-Antiplasminbindungsstelle verwendet, so z.B. Miniplasminogen.

Bei der Anwendung des erfindungsgemäßen Verfahrens zum Bestimmen der Aktivität von Serinproteasen und Serinproteaseninhibitoren kann die Umsetzung von Substraten, deren Umsetzung meßbar ist, auf zwei verschiedenen Ebenen stattfinden.
A. In bevorzugter Weise kann für die Bestimmung der Aktivität von Plasminogenaktivator-Inhibitoren und Plasminogenaktivatoren ein nichtchromogenes Plasminogenaktivator-Substrat, also Plasminogen oder jedes seiner natürlichen oder synthetischen Derivate, eingesetzt werden, das nach Aktivierung durch den verbleibenden Plasminogenaktivator die Umwandlung eines zweiten Substrates katalysiert. Für den Test sollten Lösungen mit Plasminogenkonzentrationen von 2,5 bis 20 CTA-U/ml (1 CTA-U=278 pM Plasminogen), bevorzugt zwischen 5 und 10 CTA-U/ml in einer gepufferten Lösung, enthaltend zweckmäßigerweise 50 bis 150 mM/l Tris-HCl pH 7,5 bis pH 9,5, bis zu 100 mM/l Natriumchlorid, 1 Gew.% vernetzte Polypeptide, bis zu 0,2 Gew.% ^{R}Triton x 100 und gegebenenfalls geringe Mengen (ca. 10 µg/ml) alpha₂-Antiplasmin, eingesetzt werden. Das alpha₂- Antiplasmin dient zur Neutralisierung spontan gebildeten Plasmins, vor allem während der Lagerung der Lösung. Es stört den folgenden Test nicht, da es ebenso wie das endogene alpha₂-Antiplasmin durch die Zugabe des Oxidationsmittels inaktiviert wird. In bevorzugter Weise handelt es sich bei dem Oxidationsmittel um Chloramin T in einer Konzentration von 0,5 bis 15 mM/l.Bei Verwendung von Plasminogenaktivatoren, deren plasminogenolytische Aktivität durch Zugabe von omega-Aminocarbonsäure nicht inhibiert wird, kann außerdem mindestens eine omega-Aminocarbonsäure, bevorzugt Tranexamsäure in einer Konzentration von 0,2 bis 5 mM/l, zugesetzt werden. Weiterhin sollte der Probe die Lösung eines chromogenen Plasminsubstrates, bevorzugt 2 bis 20 mM/l H-D-Val-Leu-Lys-p-Nitroanilid (pNA). 2 HCl, H-D-Phe-Tyr-Arg-ANBA . 2 HCl (ANBA = -NH-C₆H₃-p-NO₂-m-COOH) oder H-D-Nva-CHA-Lys-pNA . 2 HCl in aqua dest. oder für Messungen in linearer Kinetik in einer Lösung, welche die PA-Aktivität unterdrückt, ohne die Plasmin-Aktivität zu beeinflussen, wie z.B. 200 bis 2000 mM/l NaCl oder KCl, 100 bis 1000 mM/l CsCl oder 10 bis 100 mM/l Na₂SO₃, zugefügt werden.
B. Bei der Verwendung von Gewebe-Plasminogenaktivatoren wird, wie unter A. beschrieben, verfahren. Die plasminogenolytische Aktivität wird durch Zugabe von Fibrin, polysulfatierten Polysacchariden, Polylysin, löslichem Fibrin oder Fibrinogenfragmenten stimuliert.
C. Eine alternative Ausführungsform des erfindungsgemäßen Verfahrens für die Bestimmung von Plasminogenaktivator-Inhibitoren sieht die Verwendung eines chromogenen Plasminogenaktivator-Substrates, bevorzugt pyro-Glu-Gly-Arg-p-Nitroanilid für die Bestimmung mit Urokinase oder H-D-Ile-Pro-Arg-p-Nitroanilid für die Bestimmung mit Gewebe-Plasminogenaktivator vor. Zweckmäßigerweise werden zwischen 1 und 20 mM/l, bevorzugt 2 bis 5 mM/l eines dieser chromogenen Plasminogenaktivator-Substrate in einer Pufferlösung, enthaltend z.B. 50 bis 150 mM/l Tris-Hcl, pH 7,5 bis 9,5, 1 µM/l Aprotinin, 1 Gew.% vernetzte Polypeptide (^{R}Haemaccel), und bis zu 0,05 % ^{R}Triton x 100, zur Verfügung gestellt.

Eine wichtige Anwendung des erfindungsgemäßen Verfahrens ist zweifellos die Bestimmung der Aktivität von Plasminogenaktivator-Inhibitoren. Hierfür werden der zu untersuchenden Probe entweder ein Plasminogenaktivator, ein Substrat für den Plasminogenaktivator, dessen Umsetzung nachweisbar ist, mindestens ein Oxidationsmittel und gegebenenfalls mindestens ein Detergenz, außerdem gegebenenfalls mindestens eine omega-Aminocarbonsäure zugefügt, oder aber alternativ einen Plasminogenaktivator, ein erstes Substrat für den Plasminogenaktivator, ein zweites Substrat für das Umwandlungsprodukt des ersten Substrates, dessen Umsetzung nachweisbar ist, mindestens ein Oxidationsmittel und gegebenenfalls mindestens ein Detergenz, außerdem gegebenenfalls mindestens eine omega-Aminocarbonsäure oder gegebenenfalls lösliches Fibrin oder Fibrinogenspaltprodukte, und die Menge des in der Probe enthaltenen Plasminogenaktivator-Inhibitors wird in Abhängigkeit von der Menge des nichtinhibierten zugegebenen Plasminogenaktivators gemessen.

Die verschiedenen Plasminogenaktivator-Inhibitoren weisen unterschiedliche Empfindlichkeit gegenüber den Oxidationsmitteln auf. Dies ermöglicht die selektive Bestimmung oxidationsresistenter Plasminogenaktivator-Inhibitoren wie PAI2. Messungen der gleichen Probe einmal nach dem vorstehend beschriebenen Verfahren, zum anderen nach dem im folgenden beschriebenen Verfahren zur Bestimmung oxidationsresistenter Plasminogenaktivator-Inhibitoren, ermöglichen durch Bestimmung der Differenz zwischen dem Gesamtgehalt der Probe an Plasminogenaktivator-Inhibitoren und dem Gehalt der Probe an oxidationsresistenten Plasminogenaktivator-Inhibitoren ebenfalls die Bestimmung oxidationssensitiver Plasminogenaktivator-Inhibitoren. Für die Bestimmung oxidationsresistenter Plasminogenaktivator-Inhibitoren wie PAI2 werden der Probe zuerst mindestens ein Oxidationsmittel und gegebenenfalls mindestens ein Detergenz, außerdem gegebenfalls mindestens eine omega-Aminocarbonsäure oder eine t-PA stimulierende Substanz zugefügt, anschließend entweder ein Plasminogenaktivator, ein Substrat für den Plasminogenaktivator, dessen Umsetzung nachweisbar ist, oder aber alternativ ein Plasminogenaktivator, ein erstes Substrat für den Plasminogenaktivator, ein zweites Substrat für das Umwandlungsprodukt des ersten Substrates, dessen Umsetzung nachweisbar ist, zugefügt und die Menge des in der Probe enthaltenen oxidationsresistenten Plasminogenaktivator-Inhibitors in Abhängigkeit von der Menge des nichtinhibierten zugegebenen Plasminogenaktivators gemessen.

Das erfindungsgemäße Verfahren erlaubt auch die Quantifizierung von im Plasma vorhandenen Mengen einkettigen urinären Plasminogenaktivators (scu-PA), zweikettigen u-PA sowie ein- und zweikettigen t-PA, in dem die PA-haltigen Proben erfindungsgemäß mit omega-Aminocarbonsäure im Falle von u-PA oder eines t-PA Stimulans, Oxidationsmittel und gegebenenfalls Detergenz sowie mit definierten Mengen von Plasminogen und einem Plasminsubstrat, dessen Umsetzung nachweisbar ist, inkubiert werden. Für den Nachweis von scu-PA ist es zweckmäßig, diese in die aktivere Zweikettenform umzuwandeln. Dies kann geschehen, indem nach Plasma vor oxidation Kallikrein zugegeben oder durch Zusatz eines Oberflächenaktivators (wie Ellagsäure oder Sulfatid) erzeugt wird. Dieses Kallikrein kann - wie Plasmin - die Einketten- in die Zweikettenform der Urokinase umwandeln.

Die Bestimmung von Plasminogen wird analog der PA-Bestimmung durchgeführt. In diesem Fall wird die plasminogenhaltige biologische Flüssigkeit ebenfalls mit einem Oxidationsmittel, wie z.B. Chloramin-T und einem Detergenz behandelt, außerdem werden im Reaktionsansatz Plasminogenaktivatoren und ein Plasminsubstrat, dessen Umsetzung nachweisbar ist, zur Verfügung gestellt. Nach Inaktivierung aller spezifischen und unspezifischen Plasmininhibitoren führt die Aktivierung der zu messenden Plasminogenmenge zu einer nachfolgenden Umsetzung des detektierbaren Plasminsubstrates.

Die Erfindung wird im folgenden durch die Figuren, Beispiele und Vergleichsversuche näher erläutert.

Es zeigen:
- Fig. 1:: Elimination der alpha₂-Antiplasminwirkung im Plasmasystem.
Die Figur zeigt den Einfluß von Oxidationsmitteln und/oder omega-Aminocarbonsäuren auf die Umsetzung eines chromogenen Plasminsubstrates. Der der Figur zugrundeliegende Versuch ist im Vergleichsbeispiel 1 beschrieben.
- Fig. 2:: Elimination der alpha₂-Antiplasminwirkung im Puffersystem.
Es wird die Abhängigkeit der Umsetzung eines chromogenen Substrates in Anwesenheit definierter Mengen alpha₂-Antiplasmin, die zwischen 0 und 100 % der physiologisch vorhandenen Mengen liegen, nach Zusatz der erfindungsgemäßen Substanzen gezeigt. Der der Figur zugrundeliegende Versuch ist im Vergleichbeispiel 2 beschrieben.

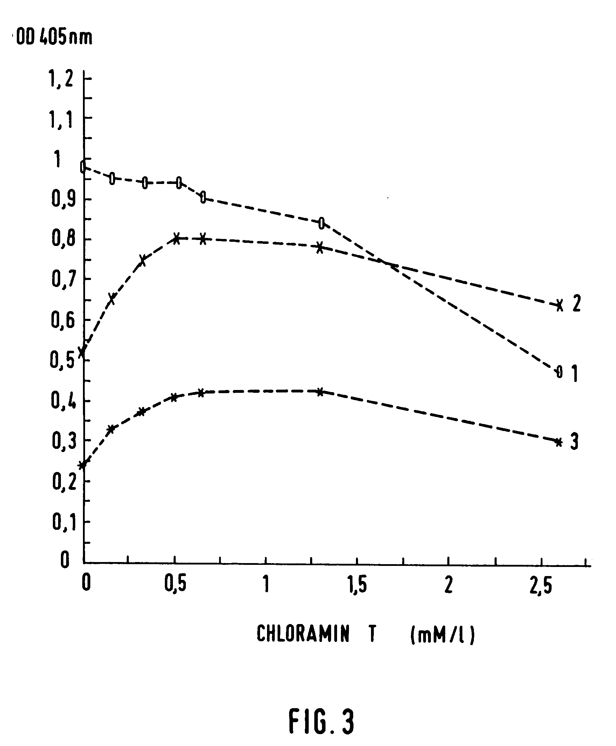

- Fig. 3:: Abhängigkeit der Stabilität des PAI/u-PA-Komplexes von der Chloramin-T-Konzentration.
Die Figur demonstriert die Stabilität des PAI/u-PA-Komplexes selbst bei Konzentrationen über 2 mmol Chloramin T/l. Der der Figur zugrundeliegende Versuch ist im Vergleichsbeispiel 3 beschrieben.

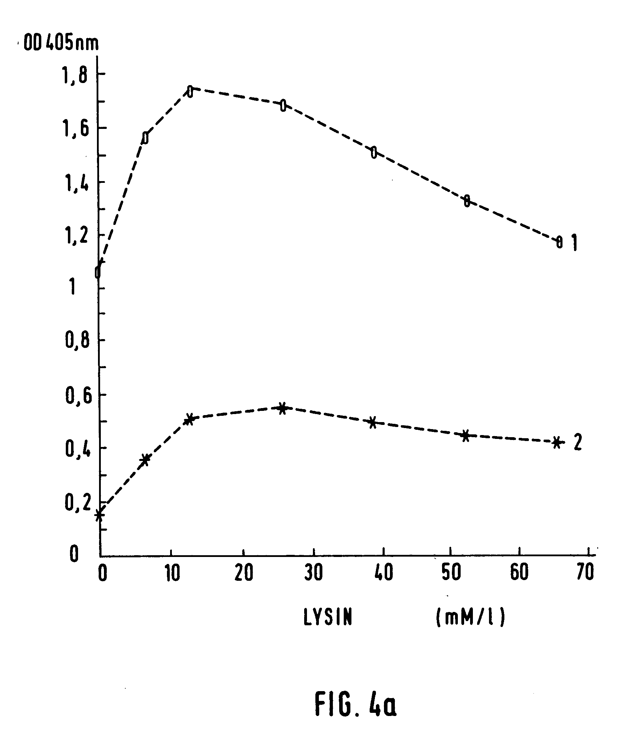

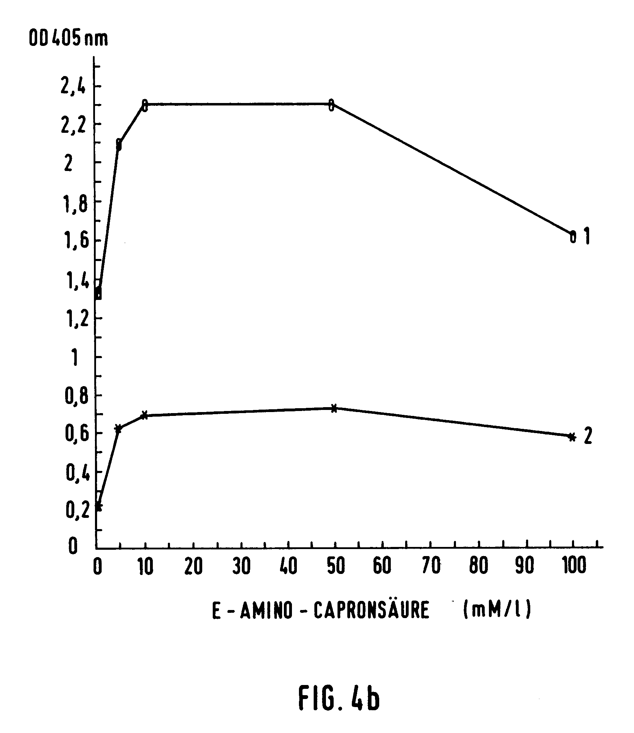

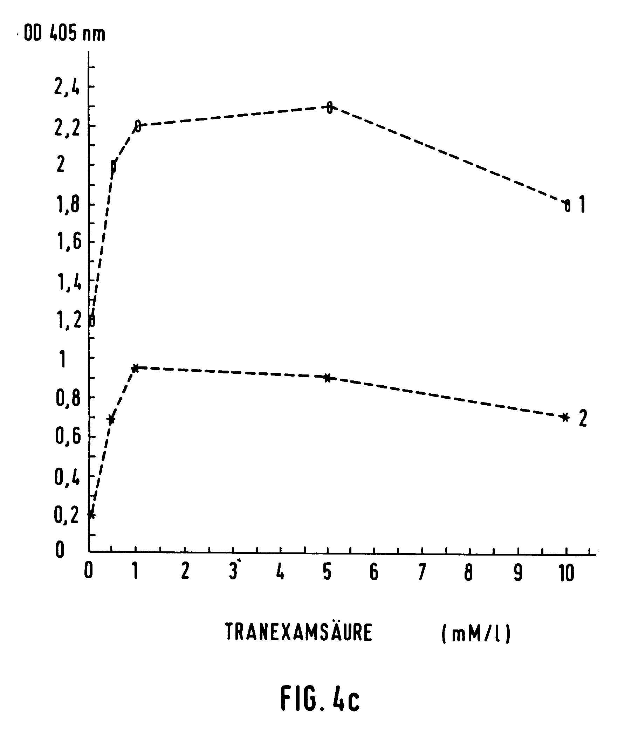

- Fig. 4:: Abhängigkeit der Plasminaktivität von der Konzentration der zugesetzten omega-Aminocarbonsäure.
Die Figuren zeigen den Einfluß verschiedener omega-Aminocarbonsäuren im erfindungsgemäßen Testsystem auf die Aktivität von Plasmin. Im einzelnen zeigt:
Fig. 4a den Einfluß von Lysin,
Fig. 4b den Einfluß von epsilon-Aminocapronsäure,
Fig. 4c den Einfluß von Tranexamsäure.
Die mit den Figuren korrelierenden Versuche sind im Vergleichsversuch 4 a-c beschrieben.

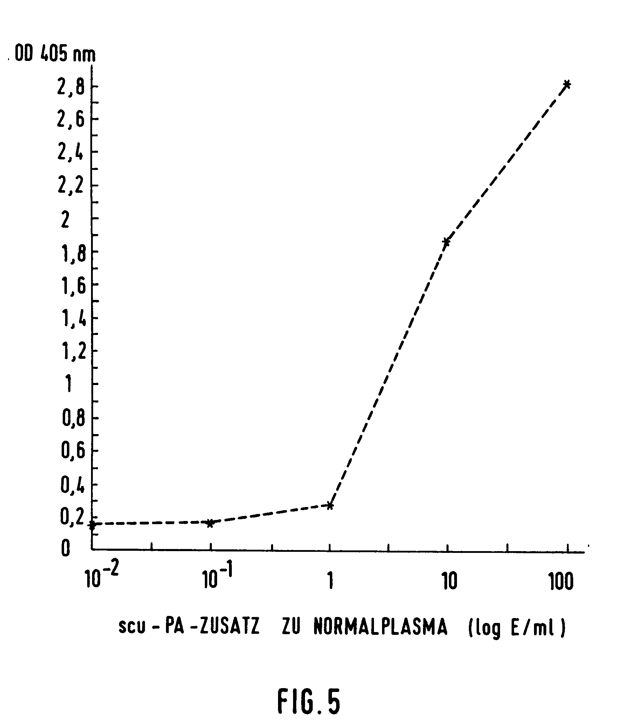

- Fig. 5:: Bestimmung von einkettigem urinären Plasminogenaktivator im Plasma.
Die Figur zeigt die Korrelation zwischen der durch Umsetzung eines chromogenen Substrates erzeugten optischen Dichte und dem scu-PA-Gehalt von supplementiertem Normalplasma. Die Durchführung des Versuches ist in Beispiel 7 beschrieben.

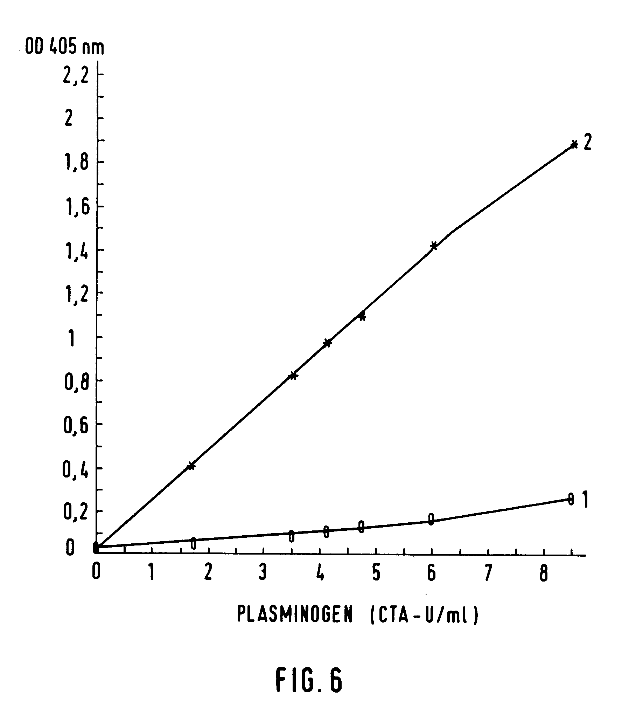

- Fig. 6:: Bestimmung der Plasminogenkonzentration im Plasma.
Die Figur zeigt den Vergleich von unbehandelten mit erfindungsgemäß mit Chloramin-T, Tranexamsäure und Detergenz behandelten Plasmaproben mit unterschiedlichem Plasminogengehalt. Der zugrundeliegende Versuch ist im Beispiel 8 beschrieben.

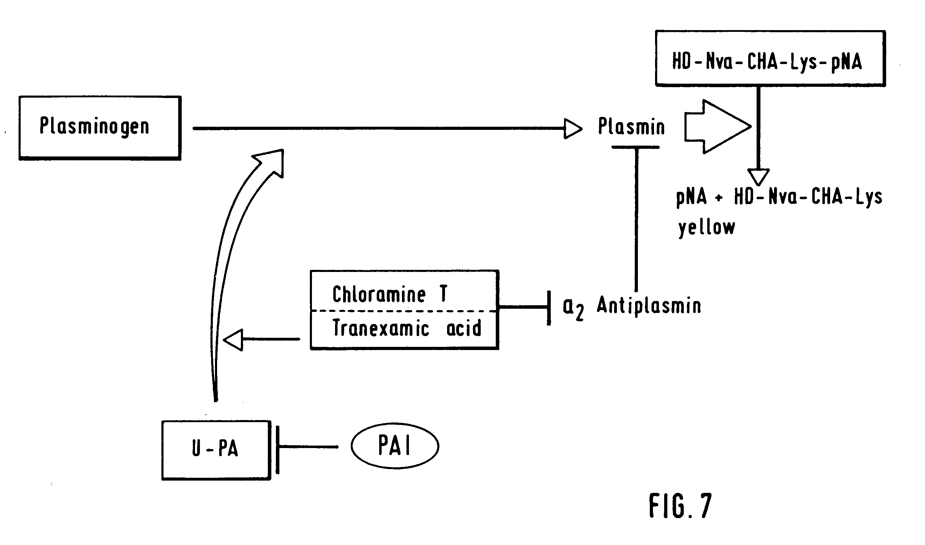

- Fig. 7:: Die Abbildung 7 gibt das Testprinzip der erfindungsgemäßen PAI (PA) Bestimmung wieder. Zum Reaktionsansatz hinzugefügte Substanzen sind umrandet dargestellt.

Die nachstehenden Beispiele und Vergleichsversuche wurden unter Verwendung der folgenden Lösungen durchgführt; die Angabe "%" bedeutet "Gew.%", wenn nicht anders angegeben.

### (A) Grundpuffer

| | |
|---|---|
| Tris-HCl pH 8,5 | 50 mM/l |
| Natriumchlorid | 100 mM/l |
| vernetzte Polypeptide | 1 % |
| ^{R}Triton x 100 | 0,05 % |
| NaN₃ | 0,01 % |

### (B) Oxidationsreagenz

| | |
|---|---|
| Chloramin T | 40 mM/l |

### (C) Plasminogenaktivator-Reagenz

| | |
|---|---|
| Urokinase | 5 IU/ml |
| Tris-HCL pH 8,8 | 50 mM/l |
| Natriumchlorid | 100 mM/l |
| vernetzte Polypeptide | 1 % |
| ^{R}Triton x 100 | 0,1 % |
| NaN₃ | 0,01 % |

### (D) Plasminogenaktivator-Substratreagenz

| | |
|---|---|
| Tris-HCl pH 8,5 | 50 mM/l |
| Aprotinin | 1 µM/l |
| vernetzte Polypeptide | 1 % |
| ^{R}Triton x 100 | 0,05 % |
| pyro-Glu-Gly-Arg-p-Nitroanilid | 2 mM/l |

Bei Verwendung von Gewebe-Plasminogenaktivatoren kann pyro-Glu-Gly-Arg-p-Nitroanilid durch H-D-Ile-Pro-Arg-p-Nitroanilid ersetzt werden.

### (E) Plasminsubstratreagenz

| | |
|---|---|
| Tris-HCl pH 8,5 | 50 mM/l |
| vernetzte Polypeptide | 1 % |
| ^{R}Triton x 100 | 0,05 % |
| H-D-Phe-Tyr-Arg-ANBA . 2 HCl | 2 mM/l |

H-D-Phe-Tyr-Arg-ANBA . 2 HCl kann wahlweise ersetzt werden durch ein anderes chromogenes Plasminsubstrat (wie z.B. H-D-Nva-CHA-Lys-pNA).

### (F) Plasminogenreagenz

| | |
|---|---|
| Tris-HCl pH 8,5 | 50 mM/l |
| vernetzte Polypeptide | 1 % |
| ^{R}Triton x 100 | 0,05 % |
| Tranexamsäure | 15 mM/l |
| Plasminogen | 20 CTA-U/ml |

Bei Verwendung oder Nachweis von Gewebe-Plasminogenaktivatoren wird Tranexamsäure durch t-PA Stimulans (1 mg/ml) ersetzt.

### (G) Grundpuffer U

| | |
|---|---|
| Tris-HCl pH 8,4 | 100 mM/l |
| vernetzte Polypeptide | 1 % |
| NaCl | 100 mM/l |
| ^{R}Triton x 100 | 0,1 % |
| NaN₃ | 0,01 % |

### Vergleichsversuch 1:

50 µl eines immunadsorptiv hergestellten alpha₂-Antiplasmin-Mangelplasmas, sowie 1:2, 1:4 und 1:8 Mischungen dieses Mangelplasmas mit Normalplasma wurden mit 200 µl Lösung C (Plasminogenaktivator-Reagenz) versetzt. Identische Proben wurden parallel mit Oxidationsmittel und/oder einer omega-Aminocarbonsäure versetzt, wie in der folgenden Tabelle angegeben:

**Tabelle 1**

| Probe | Oxidationsmittel 5 % (NH₄)₂S₂0₈ in Aquadest | omega-Aminocarbonsäure 10 % Lysin in Aquadest | H₂O |
|---|---|---|---|
| 1 | 50 µl | | 100 µl |
| 2 | | | 150 µl |
| 3 | 50 µl | 100 µl | |
| 4 | | 100 µl | 50 µl |

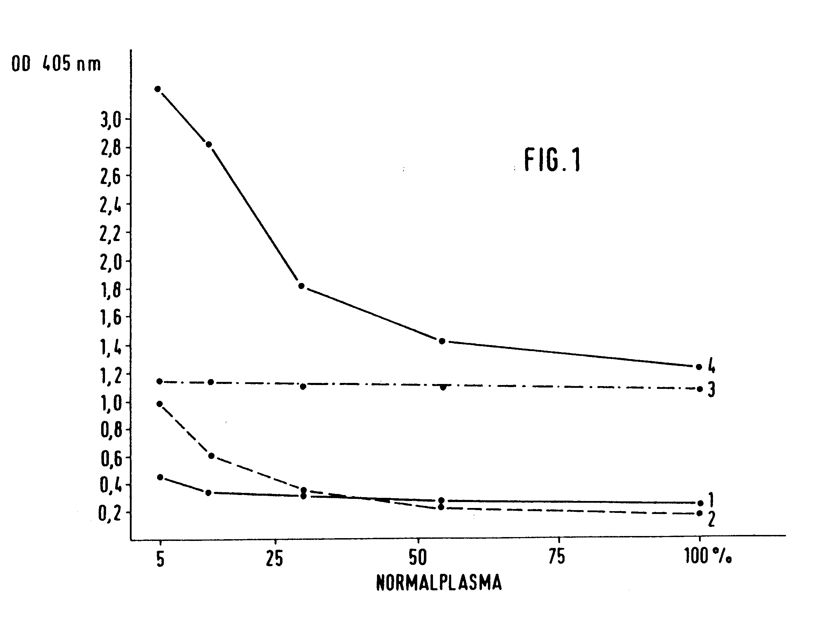

Den Proben wurden 200 µl einer 3 mM/l Lösung von H-D-Phe-Tyr-Arg-ANBA . 2 HCl in Lösung A und 500 µl einer Plasminogenlösung (2 CTA-U/ml) in Lösung A zugesetzt, die Mischungen wurden 20 min bei 37°C inkubiert, anschließend mit 100 µl 8,5 M/l Essigsäure abgestoppt und die Extinktion bei 405 nm bestimmt. Der Vergleich der Extinktionen der verschiedenen Proben ist in Fig. 1 wiedergegeben. Es wird deutlich, daß der Zusatz von Lysin die Aktivität der Urokinase um mehr als 100 % steigert. Gleichzeitig zeigt der Vergleich der Proben, daß durch die Zugabe von Ammoniumperoxodisulfat die Umsetzung des chromogenen Plasminsubstrates unabhängig von der Menge des im Plasma enthaltenen alpha₂-Antiplasmin ist.

### Vergleichsversuch 2:

### Elimination der alpha₂-Antiplasminwirkung im Puffersystem

Eine Lösung von 70 mg/l alpha₂-Antiplasmin in Lösung A (Grundpuffer), die der physiologischen Konzentration entspricht, wurde 1:1, 1:2, 1:4 und 1:8 verdünnt. 50 µl jeder Verdünnung wurden mit 200 µl Lösung C (Plasminogenaktivator-Reagenz) versetzt und anschließend wie in Vergleichversuch 1 weiterbehandelt.

**Tabelle 2**

| Probe | Oxidationsmittel 5 % (NH₄)₂S₂0₈ in Aquadest | omega-Aminocarbonsäure 10 % Lysin in Aquadest | H₂O |
|---|---|---|---|
| 1 | 50 µl | | 100 µl |
| 2 | | | 150 µl |
| 3 | 50 µl | 100 µl | |
| 4 | | 100 µl | 50 µl |

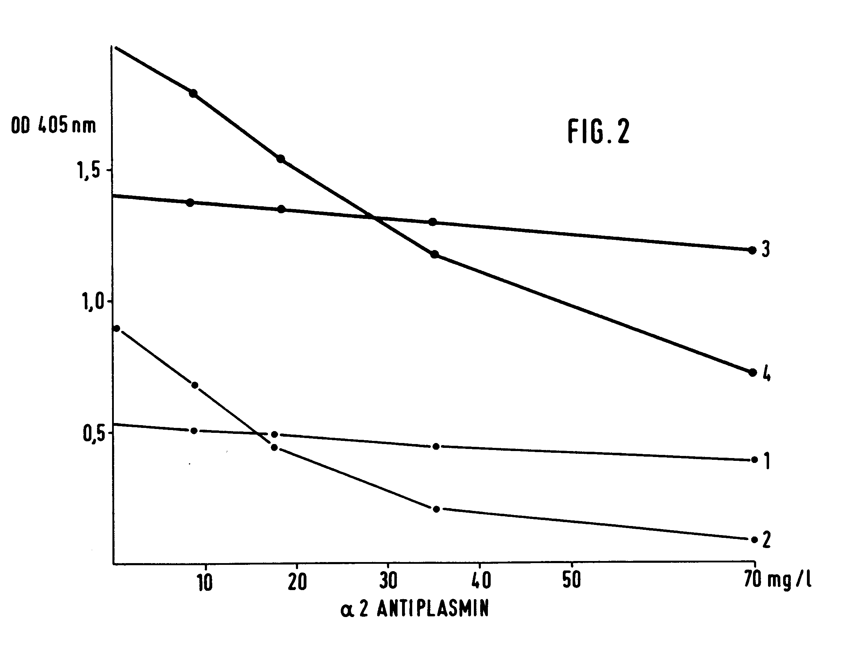

Das Ergebnis dieses Vergleichsversuches ist in Fig. 2 wiedergegeben.

### Vergleichsversuch 3:

### Abhängigkeit der Stabilität des PAI/u-PA-Komplexes von der Chloramin-T-Konzentration.

50 µl verschiedener Proben, nämlich entweder Grundpuffer A (1) oder PAI-Mangelplasma (2) und PAI-reiches Plasma (3) wurden mit 200 µl Lösung C (Plasminogenaktivator-Reagenz) 10 min bei 23°C inkubiert. Nach Zugabe von 500 µl Lösung A (Grundpuffer), 50 µl Chloramin T verschiedener Konzentration, 300 µl Lösung E (Plasminsubstrat-Reagenz) und 100 µl Lösung F (Plasminogen-Reagenz) wurde für 15 min bei 37°C inkubiert. Die Substratumsetzung wurde durch Zugabe von 100 µl 8,5 M/l Essigsäure gestoppt und die Extinktion bei 405 nm bestimmt. Das in diesem Versuch verwendete Oxidationsmittel Chloramin T wurde in Lösungen folgender Konzentrationen eingesetzt.

**Tabelle 3**

| Konzentration der Chloramin-T-Lösung (mM/l) | Chloramin-T-Konzentration im Reaktionsansatz (mM/l) |
|---|---|
| 0 | 0 |
| 3,7 | 0,15 |
| 7,5 | 0,31 |
| 15,5 | 0,64 |
| 23,5 | 0,98 |
| 31 | 1,3 |
| 62 | 2,58 |

Das Ergebnis dieses Vergleichsversuchen ist in Fig. 3 wiedergegeben. Dabei entspricht
(1) der Kurve für Grundpuffer
(2) der Kurve für PAI-Mangelplasma und
(3) der Kurve für PAI-reiches Plasma.
Aus der Abbildung ist ersichtlich, daß selbst Chloramin-T-Konzentrationen von über 2 mM/l nicht zu einer Spaltung der PAI/u-PA-Komplexe führen: die für das PAI-Mangelplasma erhaltene Kurve verläuft stets proportional zu der Kurve für PAI-reiches Plasma. Allerdings ist die Stabilität der Urokinase von der Präsenz methioninhaltiger Plasmaproteine abhängig; Urokinase im reinen Puffersystem wird bereits durch 1 mM/l Chloramin T angegriffen.

### Vergleichsversuch 4:

### Abhängigkeit der Plasminaktivität von der Konzentration der zugesetzten omega-Aminocarbonsäuren.

Zur Bestimmung des Konzentrationsbereiches, in dem verschiedene omega-Aminocarbonsäuren ihre erfindungsgemäße Wirkung zeigen, wurden verschiedene Vergleichsversuche mit
a) Lysin
b) epsilon-Aminocapronsäure
c) Tranexamsäure
nach einem einheitlichen Schema durchgeführt. Dafür wurden jeweils 50 µl Probe, und zwar entweder Lösung A (Grundpuffer) oder citratbehandeltes Plasma, mit 200 µl Lösung C (Plasminogenaktivator-Reagenz), 500 µl Lösung A (Grundpuffer), 200 µl Lösung E (Plasminsubstrat-Reagenz), 100 µl modifizierter Lösung F (Plasminogen-Reagenz), die keine omega-Aminocarbonsäure enthielt, und anschließend mit 100 µl der verschiedenen omega-Aminocarbonsäuren, entsprechend dem untenangegebenen Beispiel, versetzt. Die Mischung wurde für 20 min bei 37°C inkubiert und die Reaktion anschließend durch Zugabe von 100 µl 8,5 M/l Essigsäure abgestoppt und die Extinktion bei 405 bestimmt.
a) Vergleichsversuch für Lysin

**Tabelle 4**

| Konzentration der zugefügten Lysinlösung (mM/l) | Lysinkonzentration im Reaktionsansatz (mM/l) |
|---|---|
| 0 | 0 |
| 75 | 6,5 |
| 150 | 13 |
| 300 | 26 |
| 450 | 39 |
| 600 | 52 |
| 750 | 65 |

b) Vergleichsversuch für epsilon-Aminocapronsäure

**Tabelle 5**

| Konzentration der zugesetzten epsilon-Aminocapronsäurelösung (mM/l) | epsilon-Aminocapronsäurekonzentration im Reaktionsansatz (mM/l) |
|---|---|
| 0 | 0 |
| 55 | 4,8 |
| 110 | 9,5 |
| 550 | 47,8 |
| 1100 | 95 |

c) Vergleichsversuch für Tranexamsäure

**Tabelle 6**

| Konzentration der zugesetzten Tranexamsäure-Stammlösung (mM/l) | Tranexamsäurekonzentration im Reaktionsansatz (mM/l) |
|---|---|
| 0 | 0 |
| 5,5 | 0,48 |
| 11 | 0,95 |
| 55 | 4,8 |
| 110 | 9,5 |

Die Zeichnungen 4a bis 4c geben die Ergebnisse der Vergleichsversuche wieder. Dabei repräsentiert jeweils die Kurve 1 die für die Pufferprobe erhaltenen Werte, während die Kurve 2 die mit dem citratbehandelten Plasma erhaltene Kurve darstellt.

Die aufgrund dieser Vergleichsversuche ermittelten optimalen omega-Aminocarbonsäurekonzentrationen wurden bei der Durchführung des erfindungsgemäßen Verfahrens eingesetzt.

### Beispiel 1

### Bestimmung von Plasminogenaktivator-Inhibitor in Plasma oder Serum oder anderen biologischen Flüssigkeiten unter Verwendung eines chromogenen Urokinasesubstrats

50 µl Plasma wurden mit 200 µl Lösung C (Plasminogenaktivator-Reagenz) 10 min bei 23°C inkubiert. Nach Zugabe von 500 µl Lösung A (Grundpuffer), 50 µl Lösung B (Oxidationsreagenz) und 200 µl Lösung D (Plasminogenaktivator-Substrat-Reagenz) wurde für 200 min bei 37°C inkubiert. Die Substratumsetzung wurde durch Zugabe von 100 µl 8,5 M/l Essigsäure gestoppt und die Extinktion bei 405 nm bestimmt.

### Beispiel 2

### Bestimmung von Plasminogenaktivator-Inhibitor in Plasma oder Serum oder anderen biologischen Flüssigkeiten unter Verwendung eines chromogenen Plasminsubstrats und von Plasminogen

50 µl Plasma wurden mit 200 µl Lösung C (Plasminogenaktivator-Reagenz) 10 min bei 23°C inkubiert. Nach Zugabe von 500 µl Lösung A (Grundpuffer), 50 µl Lösung B (Oxidationsreagenz) und 200 µl Lösung E (Plasminsubstratreagenz) und 100 µl Lösung F (Plasminogenreagenz) wurde für 15 min bei 37°C oder 30 min bei 23°C inkubiert. Die Substratumsetzung wurde durch Zugabe von 100 µl 8,5 M Essigsäure gestoppt und die Extinktion bei 405 nm bestimmt.

### Beispiel 3

### Kinetische Bestimmung von Plasminogenaktivator-Inhibitor in Plasma oder Serum oder anderen biologischen Flüssigkeiten

Nach fünfminütiger Vorinkubation von 50 µl Probe mit 200 µl variierter Lösung C (Plasminogenaktivator gelöst in Grundpuffer U) bei 37°C wurden dem Reaktionsansatz 200 µl variiertes Plasminogenreagenz (F), bestehend aus 100 mM/l Tris-HCl pH 8,4, vernetzte Polypeptide 1 %, ^{R}Triton x 100 0,1 %, 100 mM/l NaCl ohne Tranexamsäure, NaN₃ 0,01 % (Grundpuffer U) mit 10 CTA-U/ml Plasminogen und 200 µl aqua dest. mit 10 mM/l Chloramin T, 3 mM/l Tranexamsäure zugesetzt. Die Mischung wurde 5 min bei 37°C inkubiert und 500 µl 0,6 mM/l chromogenes Plasminsubstrat HD-Nva-CHA-Lys-pNA in 480 mM/l NaCl (oder ersatzweise 200 mM/l CsCl) zugefügt. Die resultierende lineare Extinktionsänderung delta A/min wurde bei 405 nm verfolgt. Für Normalplasma ergaben sich Werte von ca. 0,2/min.

Alternativ zur kinetischen Messung konnte die chromogene Substratumsetzung durch Zusatz von 100 µl 8,5 M/l Essigsäure nach vier Minuten (37°C) beendet werden, woraus eine auf linearer Reaktionskinetik resultierende Endpunktbestimmung resultierte.

### Beispiel 4 a

### Bestimmung des Plasminogenaktivator-Inhibitors vom placentalen Typ (PAI2) unter Verwendung eines chromogenen Plasminogenaktivator-Substrats

50 µl Probe wurden mit 500 µl Lösung A (Grundpuffer) und 50 µl Lösung B (Oxidationsreagenz) 10 min bei 37°C inkubiert, anschließend wurde der Reaktionsansatz mit 200 µl Lösung C (Plasminogenaktivator-Reagenz) versetzt und 5 min bei 37°C inkubiert. Die Substratumsetzung wurde durch Hinzufügen von 200 µl Lösung D (Plasminogenaktivatorsubstrat-Reagenz) gestartet und die Mischung für 200 min bei 37°C inkubiert. Nach Abstoppen durch 100 µl 8,5 M/l Essigsäure wurde die Extinktion bei 405 nm bestimmt.

### Beispiel 4

### Bestimmung des Plasminogenaktivator-Inhibitors vom placentalen Typ (PAI2) unter Verwendung eines chromogenen Plasminsubstrats

50 µl Probe wurden mit 500 µl Lösung A (Grundpuffer) und 50 µl Lösung B (Oxidationsreagenz) 10 min bei 37°C inkubiert. Nach Zugabe von 200 µl Lösung C (Plasminogenaktivator-Reagenz) wurde 5 min bei 37°C inkubiert. Die Substratumsetzung wurde durch Hinzufügen von 200 µl Lösung E (Plasminsubstratreagenz) sowie 100 µl Lösung F (Plasminogenreagenz) gestartet und für 15 min bei 37°C inkubiert. Nach Zugabe von 100 µl 8,5 M/l Essigsäure wurde die Extinktion bei 405 nm bestimmt.

Die Auswertung des Versuches zeigt, daß bei der Durchführung der Oxidation vor Zugabe des Plasminogenaktivator-Reagenzes PAI1 größtenteils inaktiviert wird. Im Gegensatz dazu ist PAI2 oxidationsunempfindlicher und kann daher selektiv bestimmt werden.

### Beispiel 5 a

### Bestimmung des PAI2 über Vorinkubation mit polykationhaltigem PA-Reagenz

Testdurchführung erfolgte gemäß Beispiel 3. Unterschiedlich war lediglich der Gehalt an Protaminchlorid (100 E/ml) oder Polylysin (500 µg/ml) im Plasminogenaktivator-Reagenz, so daß ausschließlich PAI2 (und nicht PAI1) in der Messung erfaßt wird.

### Beispiel 5

### Bestimmung des Plasminogenaktivator-Inhibitors vom placentalen Typ (PAI2) über Vorinkubation mit einkettigem Gewebe-Plasminogenaktivator

50 µl Probe wurden mit 100 µl einer Lösung von 100 I.E. einkettigem Gewebe-Plasminogenaktivator in Lösung A (Grundpuffer) versetzt und 15 min bei 37°C inkubiert. Nach Zugabe von 200 µl Lösung C (Plasminogenaktivator-Reagenz) wurde wie in Beispiel 1 oder 2 verfahren. Einkettiger t-PA reagiert im Gegensatz zu PAI1 mit PAI2 sehr verlangsamt, so daß der Restgehalt an PAI (=PAI2) durch Zugabe von zweikettigem PA bestimmt werden kann.

### Beispiel 6

### Bestimmung des Plasminogenaktivator-Inhibitors vom endotheliären Typ (PAI1) unter Verwendung von Gewebe-Plasminogenaktivator und eines chromogenen Plasminsubstrats und Nachweis der Aktivität von Gewebe-Plasminogenaktivator in Plasma

50 µl Probe wurden mit 200 µl einer modifizierten Lösung C (Plasminogenaktivator-Reagenz), die anstelle der Urokinase 25 Einheiten/ml einkettigen Gewebe-Plasminogenaktivator enthielt, 15 min bei 23°C inkubiert. Nach Zugabe von 500 µl Lösung A (Grundpuffer), 20 µl Lösung B (Oxidationsreagenz), 200 µl Lösung E (Plasminsubstratreagenz), und 100 µl einer modifizierten Lösung F, die einerseits keine Tranexamsäure, andererseits durch Plasminspaltung in Anwesenheit von 5 mM/l EDTA hergestellte Fibrinogenfragmente in einer Konzentration von 1200ug/ml Lösung F enthielt, wurde 15 min bei 37°C inkubiert. Die Substratmischung wurde durch Zugabe von 100 µl 8,5 M/l Essigsäure gestoppt und die Extinktion bei 405 nm bestimmt.

Die Auswertung der Meßergebnisse aus den Beispielen 1 bis 6 erfolgte über eine Eichkurve, die durch Zugabe von Urokinase (Beispiel 1 bis 5) oder einkettigem Gewebe-Plasminogenaktivator zu Inhibitor-Mangelplasma erhalten wurde. Dieses wurde durch Zugabe von einkettigen Gewebe-Plasminogenaktivator zu Standardhumanplasma (40 I.U.(Internationale Einheiten)/ml Plasma), einstündige Inkubation bei 23°C und anschließende Immunadsorption über eine Anti-Gewebe-Plasminogenaktivator-Sepharose hergestellt. Die gemessene Plasminogenaktivator-Konzentration verhielt sich dann zur Plasminogenaktivator-Inhibitorkonzentration umgekehrt proportional.

### Beispiel 7a

### Bestimmung von Gewebe-Plasminogenaktivator in Plasma

50 µl Proben von menschlichen Plasmen, welche mit 10 bis 160 IU Einketten-t-PA supplementiert worden waren, wurden mit 100 µg eines t-PA Stimulans (wie z.B. Fibrinogenspaltprodukte durch Plasmineinwirkung in Anwesenheit von 5 mM/l EDTA) und 1 CTA-U Plasminogen und um Nullwertabgleich mit 0,6 mM (absolut) NaCl in 500 µl Grundpuffer U und 200 µl 10 mM/l Chloramin T 5 min bei 37°C inkubiert. Darauf erfolgte Zusatz von 100 µl 3 mM/l H-D-Nva-CHA-Lys-pNA in 1,2 M/l NaCl bzw. in aqua dest. (für Nullwertabgleich). Nach Inkubation über 60 min bei 37°C wurde die Substratumsetzung durch Zusatz von 100 µl 8,5 M/l Essigsäure beendet und die Extinktion bei 405 nm gemessen.

**Tabelle 7**

| Probe | Plasminaktivität Extinktion/h (mE) |
|---|---|
| Normalplasma | 0 ± 2 |
| Normalplasma + 10 IU t-PA/ml | 12 ± 1 |
| Normalplasma + 20 IU t-PA/ml | 20 ± 1 |
| Normalplasma + 40 IU t-PA/ml | 28 ± 2 |
| Normalplasma + 80 IU t-PA/ml | 60 ± 7 |
| Normalplasma + 160 IU t-PA/ml | 221 ± 12 |

Oxidative Ausschaltung des Proben-alpha₂-Antiplasmins ermöglicht die quantitative Erfassung des aktiven Gewebeplasminogenaktivators im Plasma.

### Beispiel 7

### Bestimmung von einkettigem urinärem Plasminogenaktivator (scu-PA) im Plasma

Für diesen Versuch wurden 100 u-PA-Einheiten (= 1000 ng) Scu-PA in 1 ml citratbehandeltem Standardhumanplasma aufgenommen. Durch Verdünnung mit citratbehandeltem Standardhumanplasma wurden Proben mit 100 u-PA-Einheiten/ml, 10 E/ml, 1 E/ml, 0,1 E/ml und 0,01 E/ml supplementiert. 50 µl jeder Probe wurden mit 50 µl einer 30 mM Chloramin-T- Lösung und 500 µl Lösung A 10 min bei 37°C inkubiert. Nach Zugabe von 100 µl eines chromogenen Plasminsubstrats und 200 µl einer Plasminogenlösung (2 CTA-U Plasminogen pro 200 µl einer modifizierten Lösung F, die abweichend vom Standardrezept 10 mM/l Tranexamsäure enthielt), wurde die Probe 40 min bei 37°C inkubiert. Nach Zugabe von 100 µl 8,5 M Essigsäure wurde die Extinktion bei 405 nm bestimmt.

Das Ergebnis des Versuches ist in Fig. 5 wiedergegeben.

### Beispiel 8

### Bestimmung der Plasminogenkonzentration im Plasma

Für den diesem Beispiel zugrundeliedenden Versuch wurde ein Plasminogen-Mangelplasma, das durch immunadsorptive Depletion an Antiplasminogen-^{R}Sepharose 4B hergestellt worden ist, ein citratbehandeltes Standardhumanplasma mit 3,5 CTA-U/ml Plasminogen, ein daraus durch Zusatz von Plasminogen hergestelltes hochplasminogenhaltiges Plasma mit 23,5 CTA-U/ml Plasminogen sowie Mischungen der verschiedenen Plasmen, die zu Endkonzentrationen von 1,75 CTA-U/ml, 4,13 CTA-U/ml, 4,75 CTA-U/ml, 6 CTA-U/ml, 8,5 CTA-U/ml und 13,5 CTA-U/ml führten, parallel getestet. Dafür wurden jeweils 50 µl jeder Probe mit 500 µl Lösung A (Grundpuffer), 50 µl einer 30 mM/l Chloramin-T-Lösung in H₂O, 50 µl einer 40 mM/l Tranexamsäurelösung in H₂O, 50 µl Lösung C (Plasminogenaktivator-Reagenz) mit 123 Einheiten Urokinase pro ml und 200 µl Lösung E (Plasminsubstratreagenz) 12 min bei 37°C inkubiert. Parallel wurden jeweils 50 µl der gleichen Proben mit 500 µl Lösung A (Grundpuffer), 100 µl Aquadest, 50 µl Lösung C (Plasminogenaktivator-Reagenz) mit 123 Einheiten Urokinase pro ml und 200 µl Lösung E (Plasminsubstrat-Reagenz) ebenfalls bei 37°C inkubiert, Die Reaktion aller Proben wurde anschließend durch Zugabe von 100 µl 8,5 M/l Essigsäure abgestoppt und die Extinktion bei 405 nm bestimmt.

Der Vergleich der Werte für mit Wasser behandelte Plasmen mit dem erfindungsgemäß behandelten Plasma beweist, daß ohne Zugabe der erfindungsgemäßen Substanzen praktisch keine Extinktionsänderung zu beobachten ist, d.h., keine Plasminaktivität nachweisbar ist. Im Gegensatz dazu weist die aus den erfindungsgemäß behandelten Proben resultierende Kurve eine lineare Beziehung zwischen Plasminogenkonzentrationen der Probe und Extinktion bei 405 nm aus. Daraus folgt, daß die Plasminogenkonzentration im Plasma mit dem erfindungsgemäßen Testsystem sicher bestimmt werden kann.

Das Ergebnis des Versuches ist im Fig. 6 wiedergegeben.

### Vergleichsversuch 5:

### Optimierung der Chloramin-Konzentration bei der funktionellen PAI (PA) Bestimmung

a) für Chloramin T.
   Bei der in Beispiel 3 genannten Bestimmungsmethode werden die zugesetzten Chloramin T-Konzentrationen von 0-9 mM/l Reaktionsansatz variiert. Getestet werden Proben, die a) defizient sind an funktionellem PAI (=PAI-Mangelplasma) und b) reich sind an funktionellem PAI. Man erkennt ein Optimum an generierter Plasminaktivität bei Zusatz zwischen 1 und 4 mM/l Chloramin T. Über den gesamten Bereich verlaufen beide Plasmakurven parallel, d.h. u-PA-PAI-Komplexe sind stabil bis mindestens 9 mM/l Chloramin T im Reaktionsansatz (= 650 µl).
b) für Chloramin B.
   Bei Verwendung von Chloramin B statt Chloramin T ergeben sich gleiche Ergebnisse wie für Chloramin T dargestellt.

### Vergleichsversuch 6:

### Optimierung der Tranexamsäure-Konzentration bei der funktionellen PAI (PA) Bestimmung

Grundpuffer (A) und PAI-Mangelplasma als Proben werden gemäß Beispiel 3 unter variierenden Tranexamsäure-Konzentrationen auf funktionellen PAI-Gehalt untersucht. Es ergibt sich ein Maximum bei 1-4 mM/l Tranexamsäure im Reaktionsansatz.

### Vergleichsversuch 7:

### Optimierung des Test-pH auf funktionelle PAI (PA) Bestimmung

Durch Variation des pH-Wertes des Grundpuffers U von 7,5 bis 9,5 und Bestimmung des funktionellen PAI-Gehaltes von PAI-Mangelplasma und PAI reichem Plasma gemäß Beispiel 3 zeigt sich ein pH-Optimum zwischen pH 8,3 und pH 9. Da es allerdings ab pH 8,7 zu einer Abweichung der Parallelität beider Kurven kommt, was auf Instabilitäten der u-PA-PAI-Komplexe hinweist, wird als Test-pH pH 8,4 als optimal angesehen.

### Vergleichsversuch 8:

### Optimierung der Plasminogenkonzentration bei funktioneller PAI (PA) Bestimmung

Bei variierenden Plasminogenkonzentrationen wird PAI-Mangelplasma gemäß Beispiel 3 untersucht. Glu-Plasminogen in einer Konzentration von mindestens 0,9 µM/l im Reaktionsansatz führt zum Erreichen eines Sättigungsplateaus.

### Vergleichsversuch 9:

### Optimierung der Vorinkubationszeit

PAI-Mangelplasma, Normalplasma und PAI-reiches Plasma werden gemäß Beispiel 3 bei Variation der Vorinkubationszeit untersucht. Mindestens 2 Minuten sind erforderlich für die vollständige Reaktion zwischen PAI und u-PA bei Normalplasma, bei PAI-reichem Plasma erhöht sich die notwendige Reaktionszeit auf 5 Minuten.

### Vergleichsversuch 10:

### PAI-Wiederfindungsexperimente

PAI-reiches Plasma (mit 22,5 u-PA inhibierenden Einheiten/ml) wurde mit PAI-Mangelplasma verdünnt und gemäß Beispiel 3 analysiert. Es zeigt sich, daß jede Verdünnung unter 14 u-PA inhibierenden Einheiten (E) PAI/ml zu einem direkt proportionalen linearen Anstieg der gemessenen Plasminaktivität und damit direkt proportionalen linearen Abfall der PAI-Aktivität führt. Plasmen mit mehr als 14 E PAI/ml müssen mit Plasmen bekannten, niedrigen PAI Gehaltes verdünnt werden. Unterhalb 30 % Restaktivität des eingesetzten u-PA (bei größer als 14 E PAI/ml) wird die Eichkurve unlinear und nähert sich asymptotisch der x-Achse.

### Vergleichsversuch 11:

### Einfluß von Chloramin T und/oder Tranexamsäure auf den funktionellen Test zur PAI-Bestimmung

Um zu zeigen, daß dem erfindungsgemäßen Prinzip entsprechende PAI-Bestimmungen unabhängig werden von Plasma alpha₂-Antiplasmin, werden Chloramin T und/oder Tranexamsäure zum Reaktionsansatz zugesetzt und PAI-Standardplasma, das heißt Plasmen bekannten PAI-Gehaltes, und alpha₂-Antiplasminmangelplasma gemäß Beisiel 3 auf funktionellen PAI-Gehalt untersucht. Es zeigt sich, daß Probenbestimmung ohne Zusatz von Chloramin T zu einer von alpha₂-Antiplasmingehalt der Probe abhängigen Generation von Plasminaktivität führt. Testdurchführung ohne Chloramin T und ohne Tranexamsäure resultierte in kompletter Hemmung des erzeugten Plasmins; es kann fast keine Extinktionsänderung detektiert werden.

Jedoch konnte bei alpha₂-Antiplasminmangelplasma eine Plasminaktivität beobachtet werden. Zusatz von Tranexamsäure allein führte zu meßbaren Plasminaktivitäten. Jedoch sind diese bei alpha₂-Antiplasminmangelplasma ca. 70% höher als bei Normalplasma, was Abhängigkeit vom Proben-alpha₂-Antiplasmin bedeutet. Wird lediglich Chloramin T zugestzt, resultiert 20 % erhöhte Plasminaktivität verglichen mit Normalplasma. Dieser Befund demonstriert nahezu Proben-alpha₂-Antiplasmin unabhängige Bestimmung von PAI. Eine Kombination von Tranexamsäure und Chloramin T bewirkt allgemein erhöhte Plasminaktivität (Wirkung der Tranexamsäure) und nur 5 % erhöhte Plasminaktivität bei alpha₂-Antiplasminmangelplasma (Wirkung des Choramin T). Funktionelle PAI-Bestimmung unter diesen Bedingungen ist unabhängig von der Plasma alpha₂-Antiplasminkonzentration. Dies bedeutet eine synergistische Wirkung aus Oxidans und omega-Aminocarbonsäure.

**Tabelle 8**

| Zusatz: | Probe:Normalplasma delta A /4 min | Normalplasma ohne alpha₂-Antiplasmin |
|---|---|---|
| aqua dest. | 40 mE | 217 mE |
| Chloramin T | 225 mE | 271 mE |
| Tranexamsäure | 488 mE | 829 mE |
| Chloramin T und Tranexamsäure | 910 mE | 952 mE |

### Vergleichsversuch 12:

### Methodenvergleich, Variationskoeffizient

Die erfindungsgemäße Bestimmung von PAI in Gegenwart von Oxidanten gemäß Beispiel 3b wurde mit einem kommerziell erhältlichen funktionellen PAI-Test korreliert, der auf der Ansäuerungsmethode beruht (^{R}Spectrolyse TM/pL von Biopool, Umea, Schweden). Proben von 20 freiwilligen Spendern wurden mit beiden Methoden gemessen. Beide Methoden korrelierten gut mit einem Korrelationskoeffizienten von r = 0,91. Intra- und interassay Variationskoeffizienten lagen beim erfindungsgemäßen Verfahren unter 5 %.

### Vergleichsversuch 13:

### Oxidative Inaktivierung von Antithrombin III (AT III), Oxidationsresistenz von Thrombin

50 pM AT III in 50 µl 50 mM/l Tris, 100 mM/l NaCl, 0,1 % (w/v) Triton x 100, pH 8,8 Puffer werden vorinkubiert mit steigenden Mengen an Chloramin T oder Wasserstoffsuperoxid in 50 µl aqua dest. für 15 min bei 37°C. Danach werden 3 pM humanes alpha-Thrombin, 2,5 IU Heparin und 2 KIU Aprotinin in 500 µl 100 mM/l Tris, 150 mM/l NaCl, pH 8,2 zugefügt und 5 min bei 37°C inkubiert. Nach Zugabe von 500 µl 0,4 mM HD-CHA-But-Arg-pNA wurde eine weitere Inkubation von 3 min (37°C) durchgeführt. Die Reaktion wurde beendet durch 100 µl 8,5 M/l Essigsäure und die Extinktionsänderung bei 405 nm bestimmt.

Ergebnis: Durch Zusatz von 500 nM Choramin T oder der 500fachen Dosis H₂O₂ werden 50 pM AT III inaktiviert, ohne daß Thrombin in seiner Aktivität beeinträchtigt ist.

### Vergleichsversuch 14:

### Stabilität von Serinprotease-Serinprotease Inhibitor Komplexen

a) u-PA-AT III Komplexe
   50 pM AT III und Pufferkontrolle wird inkubiert mit 1 IU Urokinase in Anwesenheit von 5 IU/Ml Heparin in 175 µl Grundpuffer A für 30 min bei 37°C. Danach werden 50 µl Chloramin T steigender Konzentration und 500 µl Grundpuffer A zugesetzt. Nach 30 min (37°C) wird die Substratreaktion gestartet durch Zugabe von 200 µl 2,5 mM/l chromogenes Plasminsubstrat HD-Phe-Tyr-Arg-ANBA und 120 pM Plasminogen in 200 µl Grundpuffer A. Nach 15 min (37°C) wurde die Reaktion durch 100 µl 8,5 M/l Essigsäure beendet und die Extinktionsänderung bei 405 nm bestimmt.
   Ergebnis: Im Vergleich zur Pufferkontrolle zeigt sich ein konstanter durch u-PA Komplexierung verursachter Aktivitätsverlust bei der Probe mit AT III und u-PA unabhängig von zugesetztem CT, das heißt u-PA-AT III-Kom- plexe sind resistent gegen Oxidation.
b) Plasmin-Antiplasmin-Komplexe
   30 pM alpha₂-Antiplasmin und Grundpuffer A (Kontrollansatz) werden mit 50 pM Plasmin in 175 µl Grundpuffer A für 5 min (37°C) inkubiert. Nach Zusatz von 500 µl Chloramin T unterschiedlicher Konzentration in Grundpuffer A wird 30 min (37°C) inkubiert und durch Zugabe von 200 µl 2,5 mM/l chromogenes Plasminsubstrat HD-Phe-Tyr-Arg-ANBA in Grundpuffer A die Nachweisreaktion gestartet. Nach 3 min (37°C) wird die Reaktion durch Zusatz von 100 µl 8,5 M/l Essigsäure beendet und die Extinktionsänderung bei 405 nm bestimmt. Auch durch Chloramin T-Konzentrationen über 5 mM/l im Reaktionsansatz wird keine zusätzliche Plasminaktivität generiert, das heißt Plasmin-Antiplasmin-Komplexe sind oxidationsresistent.

## Patentansprüche

1. Verfahren zur Bestimmung der Aktivität von Serinproteasen in einer Probe von Plasma oder anderen biologischen Flüssigkeiten dadurch gekennzeichnet, daß man der zu untersuchenden Probe mindestens ein Oxidationsmittel, daß die Wirkung von α2-Antiplasmin eliminiert, gegebenenfalls mindestens ein Detergenz zusetzt und die Serinproteaseaktivität in Gegenwart des Oxidationsmittels bestimmt.

2. Verfahren zur Bestimmung der Aktivität von Serinproteaseinhibitoren in einer Probe von Plasma oder einer anderen biologischen Flüssigkeit dadurch gekennzeichnet, daß man die zu untersuchende Probe mit einer definierten Menge einer Serinprotease inkubiert und dann die nicht-inhibierte Serinproteaseaktivität mittels des Verfahrens nach Anspruch 1 bestimmt.

3. Verfahren nach Anspruch 1 dadurch gekennzeichnet, daß die Serinprotease ein Plasminogenaktivator, Plasmin oder Thrombin ist.

4. Verfahren nach Anspruch 1, dadurch gekennzeichnet, daß als Oxidationsmittel ein Mittel, welches, vorzugsweise bei pH 7,5 bis pH 8,8, Methionin zu Methioninsulfoxid oxidiert, verwendet wird.

5. Verfahren nach Anspruch 1, dadurch gekennzeichnet, daß als Oxidationsmittel eine Substanz aus der Gruppe der reaktiven Halogenderivate, vorzugsweise Halogenoamine, -imine, -amide, -imide oder - hydroxyle einschließlich deren Salze, vorzugsweise Chloramin T, Chloramin B oder NaOCl verwendet wird.

6. Verfahren nach Anspruch 1, dadurch gekennzeichnet, daß als Oxidationsmittel Wasserstoffperoxid, Salze der Peroxomonoschwefelsäure oder der Peroxodischwefelsäure, oxidierte Glutathion, Chloramin T oder N-Chlorsuccinimid zugesetzt werden.

7. Verfahren nach Anspruch 1 dadurch gekennzeichnet, daß die Bestimmungsreaktion und die Oxidation gleichzeitig erfolgen.

8. Verfahren nach Anspruch 1 dadurch gekennzeichnet, daß zusätzlich mindestens eine omega-Aminocarbonsäure zugefügt wird.

9. Verfahren nach Anspruch 1 dadurch gekennzeichnet, daß der Probe ein Plasminogenaktivator und gegebenenfalls ein Substrat für diesen Aktivator, dessen Umsetzung gegebenenfalls nachweisbar ist, zugefügt wird.

10. Verfahren nach Anspruch 9 dadurch gekennzeichnet, daß als Substrat für den Plasminogenaktivator Plasminogen oder eines seiner natürlichen oder synthetischen Derivate, bevorzugt Miniplasminogen, zugefügt wird.

11. Verfahren nach Anspruch 9, dadurch gekennzeichnet, daß ein erstes Substrat für den Plasminogenaktivator sowie ein zweites Substrat für das Umwandlungsprodukt des ersten Substrates zugefügt werden, wobei die Umsetzung des zweiten Substrates nachweisbar ist.

12. Verfahren nach Anspruch 2 zum Bestimmen von Plasminogenaktivator-Inhibitoren, wobei man einer auf den Gehalt an Plasminogenaktivator-Inhibitoren zu untersuchenden Probe einen Plasminogenaktivator, ein Substrat für den Plasminogenaktivator, dessen Umsetzung nachweisbar ist, mindestens ein Oxidationsmittel und gegebenenfalls mindestens ein Detergenz zufügt, und die Menge des in der Probe enthaltenen Plasminogenaktivator-Inhibitors in Abhängigkeit von der Menge des nichtinhibierten zugegebenen Plasminogenaktivators mißt.

13. Verfahren nach Anspruch 2 zum Bestimmen von Plasminogenaktivator-Inhibitoren, wobei man einer auf den Gehalt an Plaminogenaktivator-Inhibitoren zu untersuchenden Probe einen Plasminogenaktivator, ein erstes Substrat für den Plasminogenaktivator, ein zweites Substrat für das Umwandlungsprodukt, dessen Umsetzung nachweisbar ist, mindestens ein Oxidationsmittel und gegebenenfalls mindestens ein Detergenz zufügt und die Menge des in der Probe enthaltenen Plasminogenaktivator-Inhibitors in Abhängigkeit von der Menge des nichtinhibierten zugegebenen Plasminogenaktivators mißt.

14. Verfahren nach Anspruch 2 zum Bestimmen von oxidationsresistenten Plasminogenaktivator-Inhibitoren, wobei man einer auf den Gehalt an oxidationsresistenten Plasminogenaktivator-Inhibitoren zu untersuchenden Probe zuerst mindestens ein Oxidationsmittel und gegebenenfalls mindestens ein Detergenz zufügt und anschließend einen Plasminogenaktivator, ein Substrat für den Plasminogenaktivator, dessen Umsetzung nachweisbar ist, zufügt und die Menge des in der Probe enthaltenen oxidationsresistenten Plasminogenaktivator-Inhibitors in Abhängigkeit von der Menge des nichtinhibierten zugegebenen Plasminogenaktivators mißt.

15. Verfahren nach Anspruch 2 zum Bestimmen von oxidationsresistenten Plasminogenaktivator-Inhibitoren, wobei man einer auf den Gehalt an oxidationsresistenten Plasminogenaktivator-Inhibitoren zu untersuchenden Probe zuerst mindestens ein Oxidationsmittel und gegebenenfalls mindestens ein Detergenz zufügt und anschließend einen Plasminogenaktivator, ein erstes Substrat für den Plasminogenaktivator, ein zweites Substrat für das Umwandlungsprodukt des ersten Substrates, dessen Umsetzung nachweisbar ist, zufügt und die Menge des in der Probe enthaltenen oxidationsresistenten Plasminogenaktivator-Inhibitors in Abhängigkeit von der Menge des nichtinhibierten zugegebenen Plasminogenaktivators mißt.

16. Verfahren nach Anspruch 1 zum Bestimmen von urinärem oder Gewebetyp-Plasminogenaktivator (PA), wobei man einer auf den Gehalt an PA zu untersuchenden Probe ein Substrat für den Plasminogenaktivator, dessen Umsetzung nachweisbar ist, mindestens ein Oxidationsmittel und gegebenenfalls mindestens ein Detergenz zufügt und die Menge des in der Probe enthaltenen PA's in Abhängigkeit von der Menge des umgesetzten nachweisbaren Substrates mißt.

17. Verfahren nach Anspruch 1 zum Bestimmen von urinärem oder Gewebetyp-Plasminogenaktivator (PA), wobei man einer auf den Gehalt an PA zu untersuchenden Probe ein erstes Substrat für den Plasminogenaktivator, ein zweites Substrat für das Umwandlungprodukt des ersten Substrates, dessen Umsetzung nachweisbar ist, mindestens ein Oxidationsmittel und gegebenenfalls mindestens ein Detergenz zufügt und die Menge des in der Probe enthaltenen PA's in Abhängigkeit von der Menge des umgesetzten nachweisbaren Substrates mißt.

18. Verfahren zum Bestimmen von Plasminogen, wobei man einer auf den Gehalt an Plasminogen zu untersuchenden Probe einen Plasminogenaktivator zusetzt und das entstehende Plasmin mit dem Verfahren nach Anspruch 1 bestimmt.

19. Verwendung eines Oxidationsmittels zur Vorbereitung von Plasma oder einer biologischen Flüssigkeit für ein Verfahren nach Anspruch 1.

20. Verfahren nach Anspruch 2 zum Bestimmen von Plasminogenaktivator-Inhibitoren plazentaren Typs (2), wobei in dem Vorinkubationsansatz Polykationen, vorzugsweise Protaminchlorid, enthalten sind.

21. Verfahren nach Anspruch 1 zum Bestimmen von urinären Einkettenformen von Plasminogenaktivatoren, wobei Kallikrein oder ein Oberflächen(Kontakt)aktivator dem Testansatz zugegeben wird.

22. Verfahren nach Anspruch 1 oder 2 dadurch gekennzeichnet, daß das Oxidationsmittel Chloramin T in einer Konzentration von 0.1 bis 25 mmol/l eingesetzt wird.

23. Verfahren nach Anspruch 1 - 3 dadurch gekennzeichnet, daß der Probe weiterhin mindestens eine Verbindung aus einer der folgenden Substanzgruppen zugesetzt wird:
(I) omega-Aminocarbonsäuren
(II) NaCl, KCl, CsCl und Na₂SO₃.

24. Verfahren gemäß Anspruch 23 dadurch gekennzeichnet, daß aus Gruppe (I) Lysin und aus Gruppe II CsCl eingesetzt werden und die Lysinkonzentration im Ansatz nach Zugabe der Probe bevorzugterweise 10 - 3000, besonders bevorzugterweise 20 - 100 mmol/l und die CsCl Konzentration bevorzugterweise 100 - 1000 mmol/l beträgt.

25. Verfahren gemäß Anspruch 23 dadurch gekennzeichnet, daß aus Gruppe (I) Tranexamsäure und aus Gruppe (II) CsCl eingesetzt werden und die Tranexamsäurekonzentration bevorzugterweise 0.1 - 50 mmol/l ist.

## Claims

1. A method for the determination of the activity of serine proteases in a sample of plasma or other biological fluids, which comprises adding at least one oxidizing agent which eliminates the effect of α2-antiplasmin and, where appropriate, at least one detergent to the sample which is to be examined, and determining the serine protease activity in the presence of the oxidizing agent.

2. A method for the determination of the activity of serine protease inhibitors in a sample of plasma or another biological fluid, which comprises incubating the sample which is to be examined with a defined amount of a serine protease and then determining the non-inhibited serine protease activity by means of the method as claimed in claim 1.

3. The method as claimed in claim 1, wherein the serine protease is a plasminogen activator, plasmin or thrombin.

4. The method as claimed in claim 1, wherein the oxidizing agent used is an agent which oxidizes methionine to methionine sulfoxide, preferably at pH 7.5 to pH 8.8.

5. The method as claimed in claim 1, wherein the oxidizing agent used is a substance from the group comprising reactive halogen derivatives, preferably haloamines, haloimines, haloamides, haloimides or halohydroxyls, including the salts thereof, preferably chloramine T, chloramine B or NaOCl.

6. The method as claimed in claim 1, wherein hydrogen peroxide, salts of peroxomonosulfuric acid or of peroxodisulfuric acid, oxidized glutathione, chloramine T or N-chlorosuccinimide are added as oxidizing agents.

7. The method as claimed in claim 1, wherein the determination reaction and the oxidation take place simultaneously.

8. The method as claimed in claim 1, wherein additionally at least one omega-amino carboxylic acid is added.

9. The method as claimed in claim 1, wherein a plasminogen activator and, where appropriate, a substrate for this activator, whose conversion can be detected where appropriate, are added to the sample.

10. The method as claimed in claim 9, wherein the plasminogen activator substrate which is added is plasminogen or one of its natural or synthetic derivatives, preferably miniplasminogen.

11. The method as claimed in claim 9, wherein a first substrate for the plasminogen activator, and a second substrate for the conversion product of the first substrate, are added, with the conversion of the second substrate being detectable.

12. The method as claimed in claim 2 for the determination of plasminogen activator inhibitors, entailing a plasminogen activator, a plasminogen activator substrate whose conversion can be detected, at least one oxidizing agent and, where appropriate, at least one detergent being added to a sample whose content of plasminogen activator inhibitors is to be examined, and the quantity of plasminogen activator inhibitor contained in the sample being measured as a function of the quantity of non-inhibited plasminogen activator which is added.

13. The method as claimed in claim 2 for the determination of plasminogen activator inhibitors, entailing a plasminogen activator, a first substrate for the plasminogen activator, a second substrate for the conversion product, whose conversion can be detected, at least one oxidizing agent and, where appropriate, at least one detergent being added to a sample whose content of plasminogen activator inhibitors is to be examined, and the quantity of plasminogen activator inhibitor contained in the sample being measured as a function of the quantity of non-inhibited plasminogen activator which is added.

14. The method as claimed in claim 2 for the determination of oxidation-resistant plasminogen activator inhibitors, entailing initially at least one oxidizing agent and, where appropriate, at least one detergent being added to a sample whose content of oxidation-resistant plasminogen activator inhibitors is to be examined, and subsequently a plasminogen activator and a plasminogen activator substrate whose conversion can be detected being added, and the quantity of oxidation-resistant plasminogen activator inhibitor contained in the sample being measured as a function of the quantity of non-inhibited plasminogen activator which is added.

15. The method as claimed in claim 2 for the determination of oxidation-resistant plasminogen activator inhibitors, entailing initially at least one oxidizing agent and, where appropriate, at least one detergent being added to a sample whose content of oxidation-resistant plasminogen activator inhibitors is to be examined, and subsequently a plasminogen activator, a first substrate for the plasminogen activator, and a second substrate for the conversion product of the first substrate, whose conversion can be detected, being added, and the quantity of oxidation-resistant plasminogen activator inhibitor contained in the sample being measured as a function of the quantity of non-inhibited plasminogen activator which is added.

16. The method as claimed in claim 1 for the determination of urinary or tissue-type plasminogen activator (PA), entailing a plasminogen activator substrate whose conversion can be detected, at least one oxidizing agent and, where appropriate, at least one detergent being added to a sample whose content of PA is to be examined, and the quantity of PA contained in the sample being measured as a function of the quantity of detectable substrate which is converted.

17. The method as claimed in claim 1 for the determination of urinary or tissue-type plasminogen activator (PA), entailing a first substrate for the plasminogen activator, a second substrate for the conversion product of the first substrate, whose conversion can be detected, at least one oxidizing agent and, where appropriate, at least one detergent being added to a sample whose content of PA is to be examined, and the quantity of PA contained in the sample being measured as a function of the quantity of detectable substrate which is converted.

18. A method for the determination of plasminogen, entailing a plasminogen activator being added to a sample whose content of plasminogen is to be examined, and the plasmin formed being determined by the method as claimed in claim 1.

19. The use of an oxidizing agent for preparing plasma or a biological fluid for a method as claimed in claim 1.

20. The method as claimed in claim 2 for the determination of plasminogen activator inhibitors of the placental type (2), entailing the preincubation mixture containing polycations, preferably protamine chloride.

21. The method as claimed in claim 1 for the determination of urinary single-chain forms of plasminogen activators, entailing the addition of kallikrein or a surface (contact) activator to the assay mixture.

22. The method as claimed in claim 1 or 2 wherein the oxidizing agent chloramine T is used in a concentration of 0.1 to 25 mmol/l.

23. The method as claimed in claims 1-3 wherein at least one compound from one of the following substance groups:
(I) omega-amino carboxylic acids
(II) NaCl, KCl, CsCl and Na₂SO₃ is additionally added to the sample.

24. The method as claimed in claim 23 wherein lysine from group (I) and CsCl from group (II) are used, and the lysine concentration in the mixture after addition of the sample is preferably 10-3000, particularly preferably 20-100, mmol/l and the CsCl concentration is preferably 100-1000 mmol/l.

25. The method as claimed in claim 23 wherein tranexamic acid from group (I) and CsCl from group (II) are used, and the tranexamic acid concentration is preferably 0.1-50 mmol/l.

## Revendications

1. Procédé pour la détermination de l'activité de sérine protéases dans un échantillon de plasma ou d'autres liquides biologiques, caractérisé en ce que l'on ajoute à l'échantillon à étudier au moins un oxydant qui élimine l'action de l'α2-antiplasmine, et éventuellement un détergent, et on détermine l'activité sérine protéase en présence de l'oxydant.

2. Procédé pour la détermination de l'activité d'inhibiteurs de sérine protéases dans un échantillon de plasma ou d'un autre liquide biologique, caractérisé en ce que l'on met à incuber l'échantillon à étudier avec une quantité définie d'une sérine protéase et on détermine ensuite au moyen du procédé selon la revendication 1 l'activité sérine protéase non inhibée.

3. Procédé selon la revendication 1, caractérisé en ce que la sérine protéase est un activateur du plasminogène, la plasmine ou la thrombine.

4. Procédé selon la revendication 1, caractérisé en ce que, comme oxydant, on utilise un agent qui oxyde la méthionine en méthionine sulfoxyde, de préférence à pH 7,5 à 8,8.

5. Procédé selon la revendication 1, caractérisé en ce que, comme oxydant, on utilise une substance choisie parmi des dérivés halogénés réactifs, de préférence des halogéno-amines, -imines, -amides, -imides ou hydroxyles, y compris leurs sels, de préférence la chloramine T, la chloramine B ou NaOCl.

6. Procédé selon la revendication 1, caractérisé en ce que, comme oxydant, on ajoute du peroxyde d'hydrogène, des sels de l'acide peroxomonosulfurique ou de l'acide peroxodisulfurique, du glutathion oxydé, de la chloramine T ou du N-chlorosuccinimide.

7. Procédé selon la revendication 1, caractérisé en ce que la réaction de détermination et l'oxydation s'effectuent simultanément.

8. Procédé selon la revendication 1, caractérisé en ce que l'on ajoute en outre au moins un acide ω-aminocarboxylique.

9. Procédé selon la revendication 1, caractérisé en ce que l'on ajoute à l'échantillon un activateur du plasminogène et éventuellement un substrat pour cet activateur, dont la réaction est éventuellement détectable.

10. Procédé selon la revendication 9, caractérisé en ce que, comme substrat pour l'activateur du plasminogène, on ajoute du plasminogène ou un de ses dérivés naturels ou synthétiques, de préférence du miniplasminogène.

11. Procédé selon la revendication 9, caractérisé en ce que l'on ajoute un premier substrat pour l'activateur du plasminogène ainsi qu'un second substrat pour le produit de conversion du premier substrat, la réaction du second substrat étant détectable.

12. Procédé selon la revendication 2, pour la détermination d'inhibiteurs d'activateurs du plasminogène, dans lequel on ajoute à un échantillon, à analyser en ce qui concerne la teneur en inhibiteurs d'activateurs du plasminogène, un activateur du plasminogène, un substrat pour l'activateur du plasminogène, dont la réaction est détectable, au moins un oxydant et éventuellement au moins un détergent, et on mesure la quantité de l'inhibiteur d'activateur du plasminogène contenu dans l'échantillon, en fonction de la quantité de l'activateur du plasminogène ajouté non inhibé.

13. Procédé selon la revendication 2, pour la détermination d'inhibiteurs d'activateurs du plasminogène, dans lequel on ajoute à un échantillon, à analyser en ce qui concerne la teneur en inhibiteurs d'activateurs du plasminogène, un activateur du plasminogène, un premier substrat pour l'activateur du plasminogène, un second substrat pour le produit de conversion, dont la réaction est détectable, au moins un oxydant et éventuellement au moins un détergent, et on mesure la quantité de l'inhibiteur d'activateur du plasminogène contenu dans l'échantillon, en fonction de la quantité d'activateur du plasminogène ajouté non inhibé.

14. Procédé selon la revendication 2, pour la détermination d'inhibiteurs d'activateurs du plasminogène, résistants à l'oxydation, dans lequel on ajoute d'abord au moins un oxydant et éventuellement au moins un détergent à un échantillon à analyser en ce qui concerne la teneur en inhibiteurs d'activateurs du plasminogène, résistants à l'oxydation, et on y ajoute ensuite un activateur du plasminogène, un substrat pour l'activateur du plasminogène, dont la réaction est détectable, et on mesure la quantité de l'inhibiteur d'activateur du plasminogène, résistant à l'oxydation, contenu dans l'échantillon, en fonction de la quantité de l'activateur du plasminogène ajouté non inhibé.

15. Procédé selon la revendication 2, pour la détermination d'inhibiteurs d'activateurs du plasminogène, résistants à l'oxydation, dans lequel on ajoute d'abord au moins un oxydant et éventuellement au moins un détergent à un échantillon à analyser en ce qui concerne la teneur en inhibiteurs d'activateurs du plasminogène, résistants à l'oxydation, et on y ajoute ensuite un activateur du plasminogène, un premier substrat pour l'activateur du plasminogène, un second substrat pour le produit de conversion du premier substrat, dont la réaction est détectable, et on mesure la quantité de l'inhibiteur d'activateur du plasminogène, résistant à l'oxydation, contenu dans l'échantillon, en fonction de la quantité de l'activateur du plasminogène ajouté non inhibé.

16. Procédé selon la revendication 1, pour la détermination de l'activateur du plasminogène (AP) du type tissulaire ou urinaire, dans lequel on ajoute à un échantillon, à analyser en ce qui concerne la teneur en AP, un substrat pour l'activateur du plasminogène, dont la réaction est détectable, au moins un oxydant et éventuellement au moins un détergent, et on mesure la quantité de l'AP contenu dans l'échantillon, en fonction de la quantité du substrat détectable qui a réagi.

17. Procédé selon la revendication 1, pour la détermination de l'activateur du plasminogène (AP) du type tissulaire ou urinaire, dans lequel on ajoute à un échantillon à analyser, en ce qui concerne la teneur en AP, un premier substrat pour l'activateur du plasminogène, un second substrat pour le produit de conversion du premier substrat, dont la réaction est détectable, au moins un oxydant et éventuellement au moins un détergent, et on mesure la quantité de l'AP contenu dans l'échantillon, en fonction de la quantité du substrat détectable qui a réagi.

18. Procédé pour la détermination du plasminogène, dans lequel on ajoute un activateur du plaminogène à un échantillon à analyser en ce qui concerne la teneur en plasminogène, et on détermine la plasmine résultante, par le procédé selon la revendication 1.

19. Utilisation d'un oxydant pour la préparation de plasma ou d'un liquide biologique pour un procédé selon la revendication 1.

20. Procédé selon la revendication 2, pour la détermination d'inhibiteurs d'activateurs du plasminogène du type placentaire (2), dans lequel des polycations, de préférence du chlorure de protamine, sont contenus dans le mélange de pré-incubation.

21. Procédé selon la revendication 1, pour la détermination de formes monocaténaires urinaires d'activateurs du plasminogène, dans lequel on ajoute au mélange d'essai de la kallikréine ou un activateur (de contact) de surface.

22. Procédé selon la revendication 1 ou 2, caractérisé en ce que l'oxydant chloramine T est utilisé à une concentration de 0,1 à 25 mmoles/l.

23. Procédé selon l'une des revendications 1 à 3, caractérisé en ce que l'on ajoute en outre à l'échantillon au moins un composé choisi parmi les groupes de substance suivants:
(I) des acides ω-aminocarboxyliques (II) NaCl, KCl, CsCl ou Na₂SO₃.

24. Procédé selon la revendication 23, caractérisé en ce que l'on utilise dans le groupe (I) la lysine et dans le groupe (II) CsCl, et la concentration de la lysine dans le mélange, après addition de l'échantillon, est de préférence de 10 - 3 000, de façon particulièrement préférée de 20-100 mmoles/l, et la concentration de CsCl est de préférence de 100 - 1 000 mmoles/l.

25. Procédé selon la revendication 23, caractérisé en ce que l'on utilise dans le groupe (I) l'acide tranexamique et dans le groupe (II) CsCl, et la concentration de l'acide tranexamique est de 0,1-50 mmoles/l.
